# EUROPEAN PATENT APPLICATION

(11) **EP 4 181 049 A1**
(43) Date of publication of application: **17.05.2023**
(21) Application number: 21843067.6
(22) Date of filing: 11.06.2021
(51) Int. Cl.: G06Q 50/10, G16H 10/60

(54) **INFORMATION PROCESSING METHOD**

(30) Priority: 13.07.2020 JP 2020120167
(71) Applicant: H.U. Group Research Institute G.K., Akiruno-shi, Tokyo 197-0833 (JP)
(72) Inventor: OMI, Kazuya, Akiruno-shi, Tokyo 197-0833 (JP); ABE, Hiroko, Akiruno-shi, Tokyo 197-0833 (JP); FUTADA, Haruhiko, Akiruno-shi, Tokyo 197-0833 (JP); KOTAKA, Takeyuki, Akiruno-shi, Tokyo 197-0833 (JP)
(74) Representative: Müller-Boré & Partner Patentanwälte PartG mbB
(86) International application number: PCT/JP2021/022382
(87) International publication number: WO 2022/014223

(57) **Abstract**

Provided is an information processing method capable of effectively using health record of each user including test results of various tests conducted at a medical institution or the like. A health record is stored in association with user identification information for each user in a database. If acquiring the user identification information read by a dispersed management system, a computer reads a health record corresponding to the acquired user identification information from the database. The computer outputs the read health record to the management system.

## Description

### [Technical Field]

The present disclosure relates to an information processing method.

### [Background Art]

In recent years, at the entrances of various facilities, the temperature of the user is measured by a thermal camera or the like to restrict the admission of the user to the facility according to the measurement result. Patent Document 1 proposes a system that permits the user to enter a facility by opening the entrance gate based on the authentication information that had been acquired indicating that the user does not carry pathogens using a pathogen sensor.

### [Prior Art Document]

### [Patent Document]

[Patent Document 1] Japanese Patent Application Laid-Open No. 2020-80083

### [Summary of Invention]

### [Problems to be Solved by Invention]

In the system disclosed in Patent Document 1, the user can detect the presence or absence of pathogens in his/her breath using a pathogen sensor installed at his/her home or a public facility or the like. Meanwhile, the user may take various tests as needed at a medical institution or the like, but the test results are only used by a doctor to diagnose the condition of a disease. Since tests conducted at a medical institution or the like are highly reliable, it is desirable that they be used effectively.

The present disclosure is made in view of such a situation, and the object is to provide an information processing method capable of effectively using the health record of the user including the test results of various tests conducted at a medical institution or the like.

### [Means for Solving Problems]

An information processing method according to one aspect of the present disclosure, wherein a health record is stored in association with user identification information for each user in a database, and the information processing method comprises processing of acquiring the user identification information read by a dispersed management system; reading from the database the health record corresponding to the user identification information acquired; and outputting the health record read to the management system.

### [Effects of Invention]

In the preset disclosure, the health record of the user including various test results conducted at a medical institution or the like can effectively be used.

### [Brief Description of Drawings]

FIG. 1 is a schematic diagram illustrating an example of the configuration of an information processing system.
FIG. 2 is a block diagram illustrating an example of the configuration of a server and a user terminal.
FIG. 3 is a schematic diagram illustrating an example of the configuration of a user DB.
FIG. 4A is a schematic diagram illustrating an example of the configuration of an event DB.
FIG. 4B is a schematic diagram illustrating an example of the configuration of a facility DB.
FIG. 4C is a schematic diagram illustrating an example of the configuration of a judgment result DB.
FIG. 5 is a block diagram illustrating an example of the configuration of an admission management server and a registration terminal.
FIG. 6 is a flowchart of one example of a registration processing procedure.
FIG. 7 is a schematic diagram illustrating an example of a screen display.
FIG. 8 is a flowchart of one example of the procedure of code issuance processing.
FIG. 9A is a schematic diagram illustrating an example of a screen display.
FIG. 9B is a schematic diagram illustrating an example of a screen display.
FIG. 9C is a schematic diagram illustrating an example of a screen display.
FIG. 10 is a flowchart of one example of a judgment processing procedure.
FIG. 11 is a flowchart of one example of a judgment processing procedure.
FIG. 12A is a schematic diagram illustrating an admission management system according to a second embodiment.
FIG. 12B is a block diagram illustrating an example of the configuration of the admission management system according to the second embodiment.
FIG. 13 is a schematic diagram illustrating an example of the configuration of a user DB according to a third embodiment.
FIG. 14A is a schematic diagram illustrating an example of the configuration of an event DB according to the third embodiment.
FIG. 14B is a schematic diagram illustrating an example of the configuration of a facility DB according to the third embodiment.
FIG. 15 is a block diagram illustrating an example of the configuration of an admission management system according to the third embodiment.
FIG. 16 is a flowchart of one example of a judgment processing procedure according to the third embodiment.
FIG. 17 is a block diagram illustrating an example of the configuration of an admission management system according to a fourth embodiment.
FIG. 18 is a flowchart of one example of a judgment processing procedure according to the fourth embodiment.
FIG. 19 is a schematic diagram illustrating an example of the configuration of a ticket DB.
FIG. 20 is a flowchart of one example of a judgment processing procedure according to a fifth embodiment.
FIG. 21 is a flowchart of one example of the procedure of code issuance processing according to a sixth embodiment.
FIG. 22 is a schematic diagram illustrating an example of a screen display.
FIG. 23 is a flowchart of one example of a judgment processing procedure according to the sixth embodiment.
FIG. 24 is a flowchart of one example of the procedure of code issuance processing according to a seventh embodiment.
FIG. 25 is a flowchart of one example of a judgment processing procedure according to the seventh embodiment.
FIG. 26 is a schematic diagram illustrating an example of the configuration of a facility DB according to an eighth embodiment.
FIG. 27 is a schematic diagram illustrating an example of a registration screen.
FIG. 28 is a flowchart of one example of a judgment processing procedure according to the eighth embodiment.
FIG. 29 is a flowchart of one example of a procedure of usage fee calculation processing.
FIG. 30 is a schematic diagram illustrating an example of a screen display.
FIG. 31 is a schematic diagram illustrating an example of the configuration of a guidance detail DB.
FIG. 32 is a flowchart of one example of a judgment processing procedure according to a tenth embodiment.
FIG. 33 is a flowchart of one example of the judgment processing procedure according to the tenth embodiment.
FIG. 34 is a schematic diagram illustrating an example of the configuration of a user DB according to an eleventh embodiment.
FIG. 35 is a flowchart of one example of a judgment processing procedure according to the eleventh embodiment.
FIG. 36A is a schematic diagram illustrating an example of the configuration of a corresponding processing DB.
FIG. 36B is a schematic diagram illustrating an example of the configuration of a passage management DB.
FIG. 37 is a flowchart of one example of a judgment processing procedure according to a twelfth embodiment.

### [Mode for Carrying Out Invention]

The following will describe in detail an information processing method according to the present disclosure with reference to the drawings depicting embodiments thereof.

### First Embodiment

An information processing system will be described that imposes passage restrictions on a user at various passing points (passing gates) using the health record of the user including various test results conducted at a medical institution or the like. The passage restrictions at the passing points include, for example, restrictions on the attendance of users at various events or restrictions on the entry of users to various facilities. The events on which attendance restrictions are imposed in the present disclosure may be various events such as concerts, exhibitions and the like. The facilities on which entry restrictions are imposed in the present disclosure may be various facilities such as schools, medical institutions, welfare facilities, public facilities, public transport facilities, theme parks, recreational facilities, facilities such as companies, stores, immigration control facilities (including drive-through checks) at national borders of airports and ports, and facilities for controlling entrance to and exit from specified areas. Also, the attendance restrictions or entry restrictions may restrict entry to buildings or premises, or may restrict entry to each room within a building. Such restrictions may only need to limit passage through a predetermined passing point (passing gate).

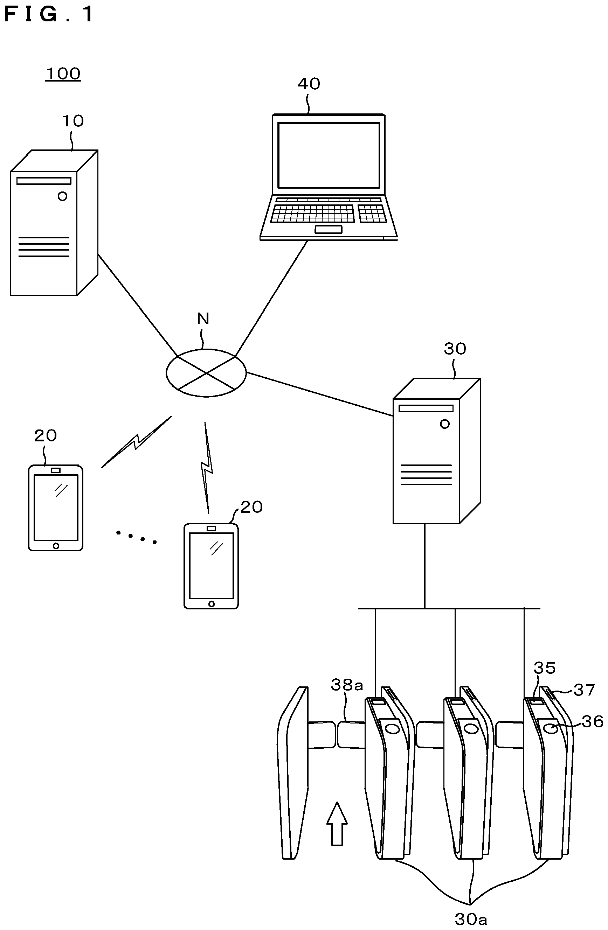

FIG. 1 is a schematic diagram illustrating an example of the configuration of an information processing system. The information processing system 100 according to the present embodiment includes a server 10, a user terminal 20, an admission management server 30, a registration terminal 40 and the like. The server 10, the user terminal 20, the admission management server 30 and the registration terminal 40 are configured to be able to communicate with each other via a network N such as the Internet.

The server 10 is a computer that manages the personal health records (PHRs) of users who have previously performed user registration. The personal health record includes, for example, the results of various laboratory tests that a user has received at a medical institution and the results of various examinations that a user has received at the time of a basic medical examination and a complete medical examination. The laboratory test includes various test items in relation to tests for the infection of infectious pathogens such as coronavirus (e.g., SARS-Cov, MER-Cov, SARS-Cov-2 (hereinafter referred to as "new coronavirus (COVID-19)"), influenza virus, hepatitis B virus (HBV), hepatitis C virus (HCV), norovirus, pneumococcus, adenovirus, and tests for allergic diseases caused by allergens such as milk (dairy products), wheat, eggs, almonds, peanuts, shrimp and crabs. Each test item also includes various tests to judge the presence or absence of antigens, antibodies or the like derived from the virus or bacteria or the like to be tested and includes, for example, an antigen test, a polymerase chain reaction (PCR) test and an antibody test. The antibody test includes, for example, a test for IgM antibody or IgG antibody against specific viruses or bacteria or for both of IgM antibody and IgG antibody, and a test for measuring the antibody content of each antibody. Furthermore, the personal health record may include medical data on a treatment history, a surgery history, a rehabilitation history and a medication history received at a medical institution or the like, the type and amount of drugs the user is taking, the frequency and amount of cigarette and alcohol being consumed, and information (genetic information) on the past medical history (history of disease, allergy or the like) of the user and his/her family. In addition, the personal health record may include a history of vaccinations to prevent the infection of various infectious diseases. The history of vaccinations may include information such as the type of a vaccine (formulation name, manufacturer name, lot number and the like), the number of vaccinations, the time of vaccination (date of vaccination), the elapsed time from the vaccination, information on the medical institution where the user has received a vaccine, in addition to the information on the presence or absence of a vaccination. Though the health record does not include simple physical information such as the height, age and weight of an individual, such physical information may be registered additionally.

The server 10 is an information processing device capable of performing various information processing and transmission and reception of information and is formed of a server computer, a personal computer or the like. The server 10 may be configured to include multiple servers for performing dispersed processing, may be implemented by multiple virtual machines provided in a single server, or may be implemented using a cloud server. The user terminal 20 is a terminal used by a user whose personal health record is managed by the server 10. The user terminal 20 is formed of an information processing device such as a smartphone, tablet terminal, a personal computer or the like or may be formed of a dedicated terminal. The admission management server 30 is composed of, for example, a server computer or a personal computer. The admission management server 30 may be configured to include multiple servers for performing dispersed processing, may be implemented by multiple virtual machines provided in a single server, or may be implemented using a cloud server. The admission management server 30 is configured to be communicable with the gate devices 30a, which are dispersedly located in places where attendance restrictions or entry restrictions (passage restrictions) are imposed on users. The gate devices 30a is each installed at the entrance to the building of the facility, the entrance to the premises, the entrance to each room in the building and the like. The admission management server 30 and the gate devices 30a constitute a management system (admission management system) that manages the attendance at the event or entry to the facility (passing through a passing point) of the user. Though the admission management server 30 is configured to control the operation of the multiple gate devices 30a in the present embodiment, the gate devices 30a may each be configured to have a function of the admission management server 30. The registration terminal 40 is used by the organizer of an event for which attendance restrictions are employed using the admission management system, or used by a person in charge of the facility for which entry restrictions are employed. The registration terminal 40 is formed of an information processing device such as a personal computer, a tablet terminal, a smartphone or the like.

In the information processing system 100 according to the present embodiment, a user who wishes to attend various events or wishes to enter various facilities has received a code including identification information of the user from the server 10 through the user terminal 20. In response to the request from the user terminal 20, the server 10 generates a code including the identification information of the user and sends it to the user terminal 20. When attending an event or entering a facility, the user displays the code on the user terminal 20 for a code reader 36 of the gate device 30a to read the code. If acquiring the identification information of the user by reading the code through the code reader 36, the admission management server 30 requests the server 10 to judge whether or not the user is permitted to attend the event or to enter the facility based on the identification information of the user. In response to the request from the admission management server 30, the server 10 judges whether or not the user is permitted to attend the event or to enter the facility based on the personal health record of the user, and sends the result of the judgment to the admission management server 30. Thus, the server 10 can judge whether or not the user is permitted attendance or entry based on the identification information of the user read from the user terminal 20 by the admission management server 30 when the user attends an event or enters a facility, and the admission management server 30 can obtain the result of the judgment. It is noted that the organizer of an event who employs attendance restrictions registers requirements for restricting attendance at the event in advance on the server 10 using the registration terminal 40, and the person in charge of a facility who employs entry restrictions registers requirements for restricting entry to the facility in advance on the server 10 using the registration terminal 40.

In the present embodiment, the case where a user attends an event means a case where a user who attends a variety of events such as a concert, an exhibition, etc., enters an event venue or concert venue. In addition, the case where a user enters a facility may correspond to a case where a child, a pupil or a student goes to school, a case where a patient or the like enters a building of a medical institution, a welfare institution or the like, a case where a user of a public transportation such as an airplane and the bullet train called Shinkansen, passes through the boarding gate at an airport or the ticket gate at a station, a case where an employee of a company enters a company building or the room, or a case where a customer enters a restaurant or the like. In addition, a case where a user enters various facilities such as a public facility, a theme park, an amusement facility or the like, a case where a user enters or leaves a country at a national border, and a case where a user enters or leaves a specified area can be possible cases for restrictions.

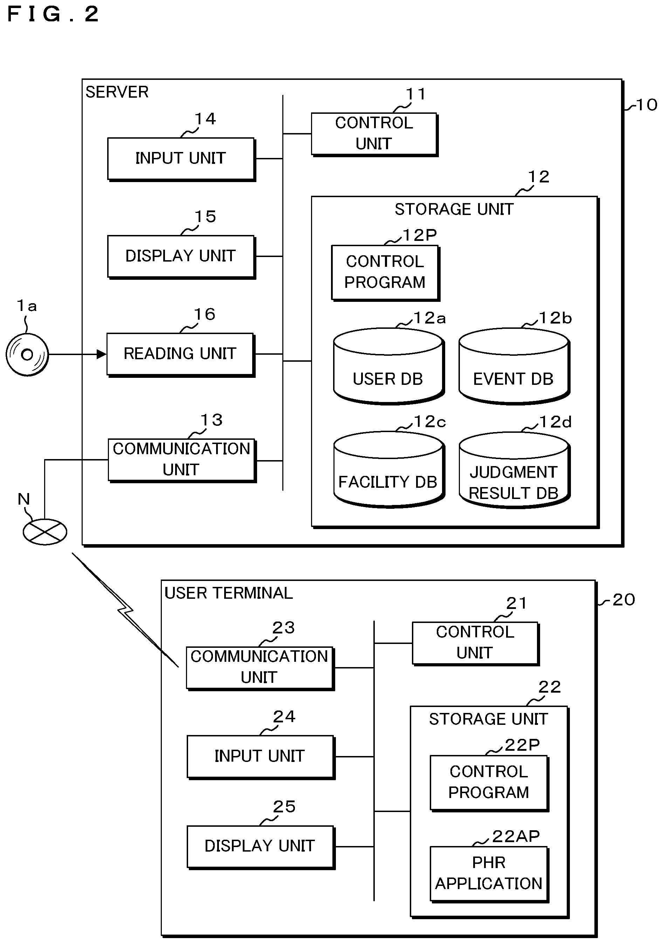

FIG. 2 is a block diagram illustrating an example of the configuration of the server 10 and the user terminal 20. The server 10 includes a control unit 11, a storage unit 12, a communication unit 13, an input unit 14, a display unit 15 and a reading unit 16 and the like that are connected to each other via a bus. The control unit 11 includes one or more processors such as a Central Processing Unit (CPU), a Micro-Processing Unit (MPU), a Graphics Processing Unit (GPU) or the like. The control unit 11 performs various information processing, control processing or the like to be performed by the server 10 by appropriately executing a control program 12P stored in the storage unit 12.

The storage unit 12 includes a Random-Access Memory (RAM), a flash memory, a hard disk, a Solid-State Drive (SSD) or the like. The storage unit 12 previously stores the control program 12P to be executed by the control unit 11 and various data or the like needed for execution of the control program 12P. Moreover, the storage unit 12 temporarily stores data or the like generated when the control unit 11 executes the control program 12P. The storage 12 also stores a user DB (database) 12a, an event DB 12b, a facility DB 12c and a judgment result DB 12d, as described below. The user DB 12a, the event DB 12b, the facility DB 12c and the judgment result DB 12d may be stored in another storage device connected to the server 10, may be stored in another storage device with which the server 10 can communicate over the network N, or may consistently be stored in other multiple servers to which the server 10 can connect in a distributed ledger format such as a blockchain.

The communication unit 13 is an interface for connecting to the network N by means of wired or wireless communication, and sends and receives information to and from other devices via the network N. The input unit 14 accepts an operation input from the user who uses the server 10, and delivers a control signal corresponding to the details of the operation to the control unit 11. The display unit 15 is a liquid crystal display, an organic EL display or the like and displays various types of information according to instructions from the control unit 11. The input unit 14 and the display unit 15 may be a touch panel constructed as one piece.

The reading unit 16 reads information stored on a portable storage medium 1a, including CD (Compact Disc)-ROM, DVD (Digital Versatile Disc)-ROM, USB (Universal Serial Bus) memory and SD (Secure Digital) cards. The control program 12P and various data stored in the storage unit 12 may be stored in the storage unit 12 by the control unit 11 reading them from the portable storage medium 1a through the reading unit 16. The control program 12P and the various data stored in the storage unit 12 may be stored in the storage unit 12 by the control unit 11 downloading them from another device through the communication unit 13.

The user terminal 20 includes a control unit 21, a storage unit 22, a communication unit 23, an input unit 24 and a display 25 that are connected to each other via a bus. The control unit 21, the storage unit 22, the communication unit 23, the input unit 24 and the display unit 25 of the user terminal 20 respectively have similar configurations to the control unit 11, the storage unit 12, the communication unit 13, the input unit 14 and the display unit 15 of the server 10, and thus the detailed descriptions will not be repeated. It is noted that the storage unit 22 of the user terminal 20 stores a PHR application program 22AP (hereinafter referred to as PHR application 22AP) for viewing a personal health record managed by the server 10 in addition to the control program 22P to be executed by the control unit 21.

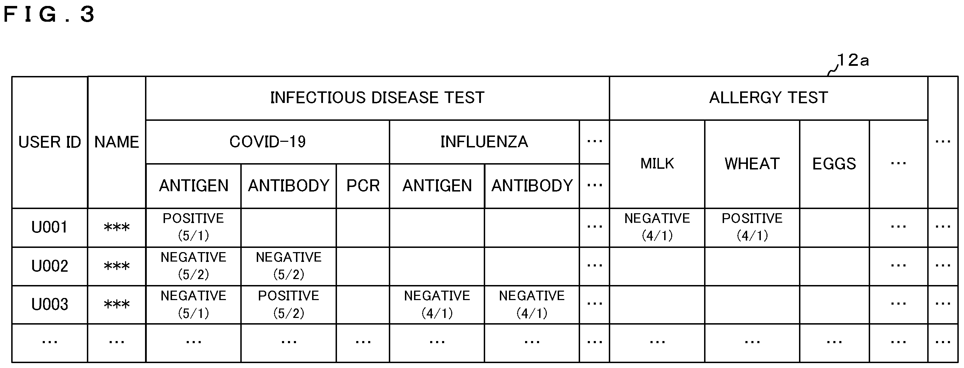

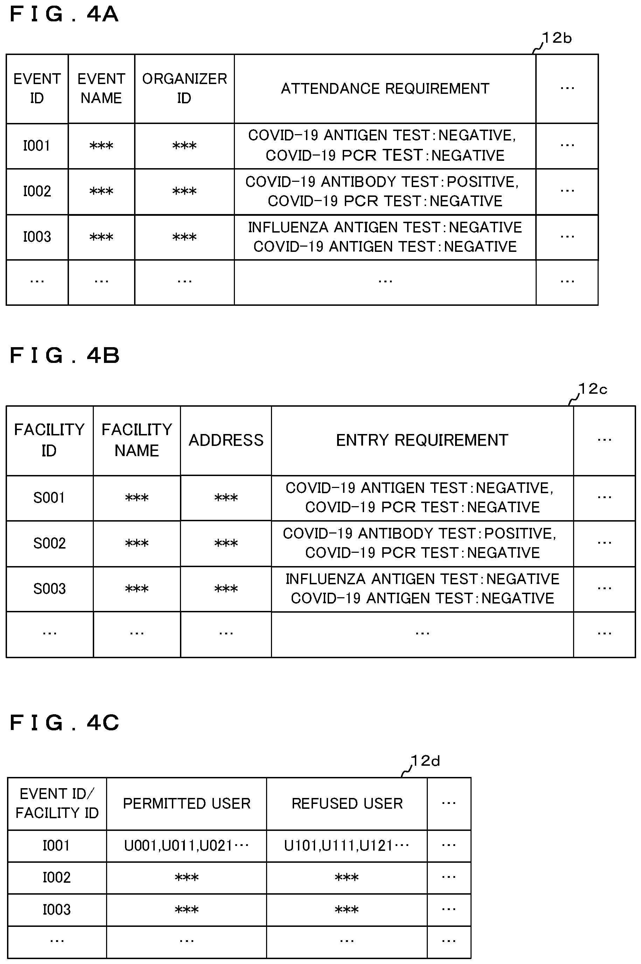

FIG. 3 is a schematic diagram illustrating an example of the configuration of the user DB 12a. FIG. 4A is a schematic diagram illustrating an example of the configuration of the event DB 12b. FIG. 4B is a schematic diagram illustrating an example of the configuration of the facility DB 12c. FIG. 4C is a schematic diagram illustrating an example of the configuration of the judgment result DB 12d. The user DB 12a stores various information including personal health record for each user who has been registered. The user DB 12a shown in FIG. 3 includes a user ID column, a name column, an infectious disease test column, an allergy test column and the like. The user ID column stores the user identification information (user ID) assigned to each registered user, and the name column stores, in association with the user ID, the name of the user designated at the time of the user registration. The infectious disease test column stores, in association with the user ID, the results of tests the user has taken for infectious diseases in order to judge whether or not the user is infected. Pathogens of the infectious diseases include a coronavirus, an influenza virus, a hepatitis B virus, a hepatitis C virus, a norovirus, a pneumococcus and an adenovirus, for example. The infectious disease test column stores the results of various tests such as an antigen test, an antibody test and a PCR test and the date of the test for each of the infectious diseases. The result of the antibody test may include, for example, the presence or absence of IgM and/or IgG antibody against viruses or bacteria to be tested and a result of measurement of the antibody content for each antibody. The allergy test column stores, in association with the user ID, the results of tests the user has taken in order to judge the presence or absence of allergic diseases. Allergens include, for example, milk, wheat, eggs, almonds, peanuts, shrimp and crabs. The test result and test date are stored for each of the allergens. The user ID stored in the user DB 12a is issued and stored by the control unit 11 when a new user is registered. The name stored in the user DB 12a is stored by the control unit 11 when the control unit 11 obtains the name of a new user through the communication unit 13 or the input unit 14. The results of the infectious disease test and the allergy test stored in the user DB 12a are stored by the control 11 when the control unit 11 obtains the results of the respective tests through the communication unit 13 or the input unit 14. The results of the infectious disease tests and the allergy tests may be deleted by the control unit 11 if a predetermined expiration period has elapsed from the test date, for example. Having been provided with expiration flags in association with the results of the infectious disease test and the allergy test in the user DB 12a, the control unit 11 may be configured to change the expiration flag corresponding to each of the test results from 0 to 1 if a predetermined expiration period has elapsed from the test date. The expiration period is set for each test item in advance and stored in the storage unit 12. Examples of the stored contents of the user DB 12a may include a variety of information on the user, not limited to the examples in FIG. 3. For example, biometric information such as the height, weight and body fat ratio of the user, medical data about treatment, surgery and rehabilitation received at a medical institution, the kind and amount of drugs the user is taking, the frequency and amount of cigarette and alcohol, the past medical history of the user and his/her family and a vaccination history may be stored in the user DB 12a. If the vaccination history is stored, the vaccination column stores information on the vaccination history of the user in association with the user ID. The types of vaccines include vaccines against coronavirus, influenza virus, hepatitis B virus, poliovirus, and pneumococcus, for example. For each type of vaccine (formulation name), vaccinated date (vaccinated period), vaccine information on the received vaccine, and information on the medical institutions where vaccinations are given are stored. The vaccine information includes, for example, a manufacturer name, a lot number and the number of vaccinations (how many doses received in the past). The information on inoculation institutions includes a name and a location (e.g., country, prefecture) of a medical institution or the like. The information on vaccination may be deleted by the control unit 11 after a predetermined expiration period has elapsed from the vaccinated date, for example. Having been provided with expiration flags in association with each of the vaccines in the user DB 12a, the control unit 11 may be configured to change the expiration flag corresponding to each of the vaccines from 0 to 1 if a predetermined expiration period has elapsed from the vaccinated date.

The event DB 12b stores information on an event registered by the event organizer for each event. The event DB 12b shown in FIG. 4A includes an event ID column, an event name column, an organizer ID column and an attendance requirement column and the like. The event ID column stores the event identification information (event ID, passing point identification information) assigned to each of the registered events. The event name column, organizer ID column and attendance requirement column respectively store the name of an event designated when the event is registered, the identification information (organizer ID) assigned to an organizer of the event and a requirement presented when an attendance at the event is permitted, in association with the event ID. The attendance requirement employs the test results for various test items. In the example in FIG. 4A, a requirement using multiple test items that the result of an antigen test for COVID-19 is negative and the result of a PCR test for COVID-19 is negative is set for the event with an event ID of I001. Although not illustrated, the presence or absence of a vaccination (within a predetermined period) and the number of vaccinations may be set as a requirement. The event ID stored in the event DB 12b is issued and stored by the control unit 11 when a new event is registered. The rest of the information stored in the event DB 12b is stored by the control unit 11 if the control unit 11 acquires the information on a new event through the communication unit 13 or the input unit 14, and changed by the control unit 11 if a change instruction is acquired. Examples of the stored contents of the event DB 12b may include a variety of information on an event, not limited to the examples shown in FIG. 4A. For example, the date and time of the event, the starting date and time of admission to the event venue, etc. may be stored. In addition, the expiration period of the test uniquely specified by the organizer may be stored as a requirement for attending the event and used in the process of judging whether or not attendance is permitted as described below.

The facility DB 12c stores information on a facility registered by a person in charge of the facility for each facility. The facility DB 12c shown in FIG. 4B includes a facility ID column, a facility name column, an address column, an entry requirement column and the like. The facility ID column stores facility identification information (facility ID and passing point identification information) assigned to each of the registered facilities. The facility name column, address column and entry requirement column respectively store, in association with the facility ID, the name of the facility, the address of the facility and the requirement presented when entry to the facility is permitted that are designated at the time of registering the facility. The entry requirement employs a requirement using test results for various test items. In the example in FIG. 4B, a requirement using multiple test items that the result of an antigen test for the influenza virus is negative and the result of an antigen test for COVID-19 is negative is set for the facility with the facility ID S003. Although not illustrated, the presence or absence of vaccinations (within a predetermined period) or the number of vaccinations may also be set as a requirement. The facility ID stored in the facility DB 12c is issued and stored by the control unit 11 when a new facility is registered. The rest of the information stored in the facility DB 12c is stored by the control unit 11 if the control unit 11 acquires the information on a new facility through the communication unit 13 or the input unit 14, and changed by the control unit 11 if a change instruction is acquired. Examples of the stored contents of the facility DB 12c may include a variety of information on the facility, not limited to the examples shown in FIG. 4B. For example, the date and time, the day of the week and the time of day when entry to the facility is permitted may be stored.

The judgment result DB 12d stores the user IDs of users who are permitted to attend the event or enter the facility and users who are refused to attend the event or enter the facility. The judgment result DB 12d shown in FIG. 4C includes an event ID/facility ID column, a permitted user column, a refused user column and the like. The event ID/facility ID column stores an event ID of each event or a facility ID (passing point identification information) of each facility. The permitted user column stores the user ID of a user who is permitted to attend the event or enter the facility, and the refused user column stores the user ID of a user who is refused to attend the event or enter the facility, in association with the event ID/facility ID. The refused user column may be configured to store the number of refused users instead of storing their user IDs. For the event ID/facility ID stored in the judgment result DB 12d, if new event information is registered in the event DB 12b or if new facility information is registered in the facility DB 12c, the event ID registered in the event DB 12b or the facility ID registered in the facility DB 12c is stored by the control unit 11. For a permitted user stored in the judgment result DB 12d, if each user is permitted to attend the event or enter the facility, the user ID of the permitted user is stored by the control unit 11, while for a refused user, if each user is refused to attend the event or enter the facility, the user ID of the refused user is stored by the control unit 11. The stored contents in the judgment result DB 12d are not limited to the examples in FIG. 4C.

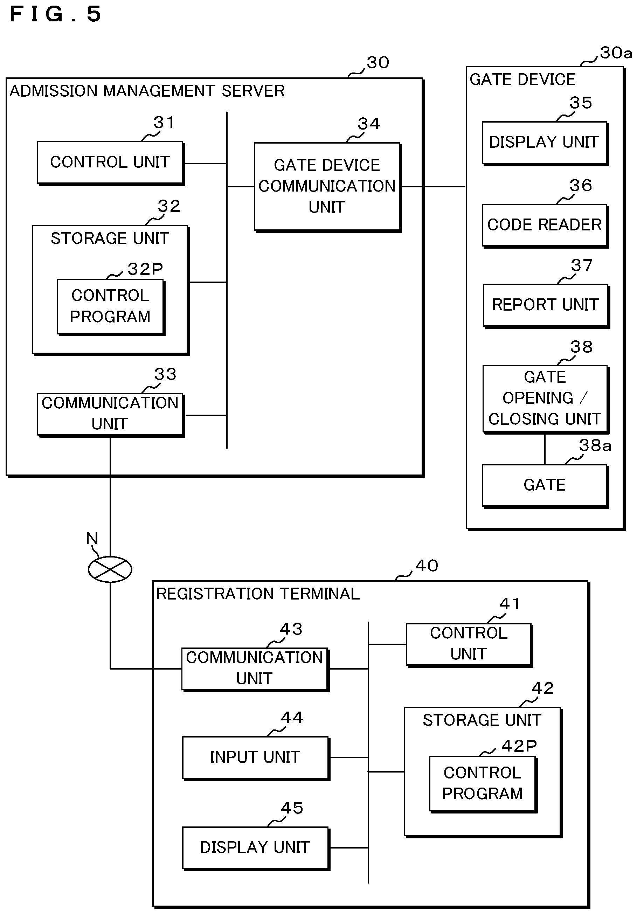

FIG. 5 is a block diagram illustrating an example of the configuration of the admission management server 30 and the registration terminal 40. The admission management server 30 includes, for example, a control unit 31, a storage unit 32, a communication unit 33 and a gate device communication unit 34 that are connected to each other via a bus. The control unit 31, the storage unit 32 and the communication unit 33 of the admission management server 30 respectively have similar configurations to the control unit 11, the storage unit 12 and the communication unit 13 of the server 10, and thus the detailed descriptions will not be repeated. The gate device communication unit 34 is connected to the gate device 30a, and the control unit 31 controls the operation of respective components of the gate device 30a through the gate device communication unit 34. The gate device 30a includes a display unit 35, a code reader 36, a report unit 37 and a gate opening/closing unit 38. The display unit 35 has a similar configuration to the display unit 15 of the server 10, and thus the detailed description will not be repeated.

The code reader 36 is a device that reads a one-dimensional code such as a bar code and a two-dimensional code such as a QR Code^{®} or is an RF tag that obtains information by radio waves (wireless communication) such as radio frequency identifier (RFID) and decodes the read code to obtain code information. The code reader 36 sequentially sends the code information read from the code to the control unit 31.

The report unit 37 is a light output unit that outputs light such as LED (light emitting diode) light or rotary light, or a sound output unit such as a buzzer or a speaker. The report unit 37 may make a report by lighting or flashing of the light output unit or by ringing of a buzzer or a voice output of the speaker. By executing predetermined report processing in accordance with an instruction from the control unit 31, the report unit 37 reports the user who passes through the gate device 30a that entry to the facility (attendance at the event) is permitted or refused. It is noted that the report unit 37 may be configured to perform the report processing by displaying various types of information on the display unit 35.

The gate opening/closing unit 38 controls the opening and closing of the gate 38a for blocking the user from proceeding through the gate device 30a in accordance with an instruction from the control unit 31. The gate opening/closing unit 38 encourages the user to proceed by opening the gate 38a and prevents the user from proceeding by closing the gate 38a, in accordance with instructions from control unit 31. In the gate device 30a of the present embodiment, an entrance passage to a facility is formed by proceeding in the direction indicated by the white arrow illustrated in FIG. 1, while the gate 38a is installed at the exit of the entrance passage. In addition, in the gate device 30a illustrated in FIG. 1, the code reader 36 is installed on the right viewed in the direction of travel of the user at the entrance side of the entrance passage, and the display unit 35 and the report unit 37 are installed at the exit side of the entrance passage. The configuration of the gate device 30a is not limited to that in FIG. 1.

The registration terminal 40 includes a control unit 41, a storage unit 42, a communication unit 43, an input unit 44 and a display 45 that are connected to each other via a bus. The control unit 41, the storage unit 42, the communication unit 43, the input unit 44 and the display unit 45 of the registration terminal 40 respectively have similar configurations to the control unit 11, the storage unit 12, the communication unit 13, the input unit 14 and the display unit 15 of the server 10, and thus the detailed descriptions thereof will not be repeated.

The processing performed by each of the devices in the information processing system 100 according to the present embodiment is described using a flowchart below. It is noted that when a user undergoes various tests at a medical institution or the like, he/she reports his/her user ID having registered in the server 10 to the medical institution. Then, a doctor or a laboratory technologist registers the test results of the various tests in association with the user ID on the server 10 using a terminal (not shown) of the medical institution, for example. This allows the results of various tests conducted at a medical institution or the like to be registered in the server 10. That is, it is assumed that the results of various tests performed for each of the users have already been registered in the user DB 12a of the server 10. The user himself/herself may register his/her own test results on the server 10 using the user terminal 20.

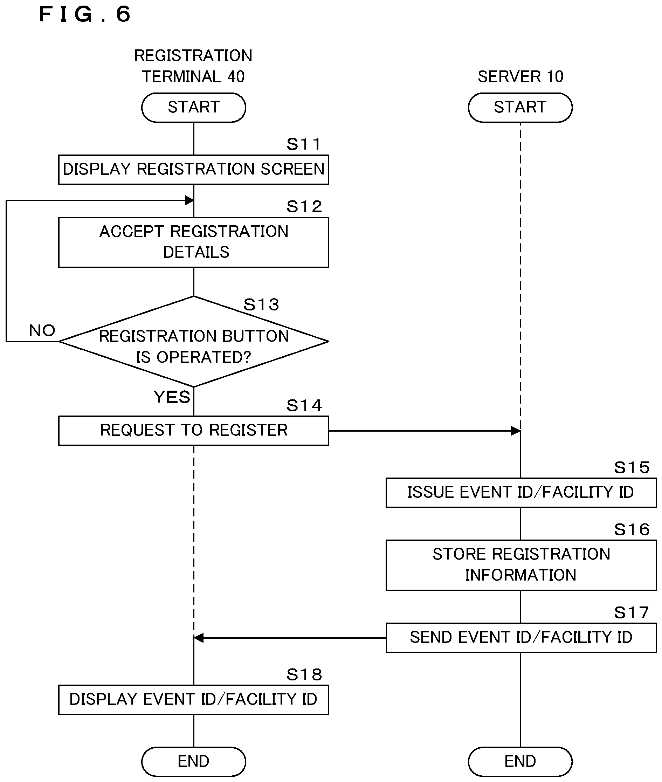

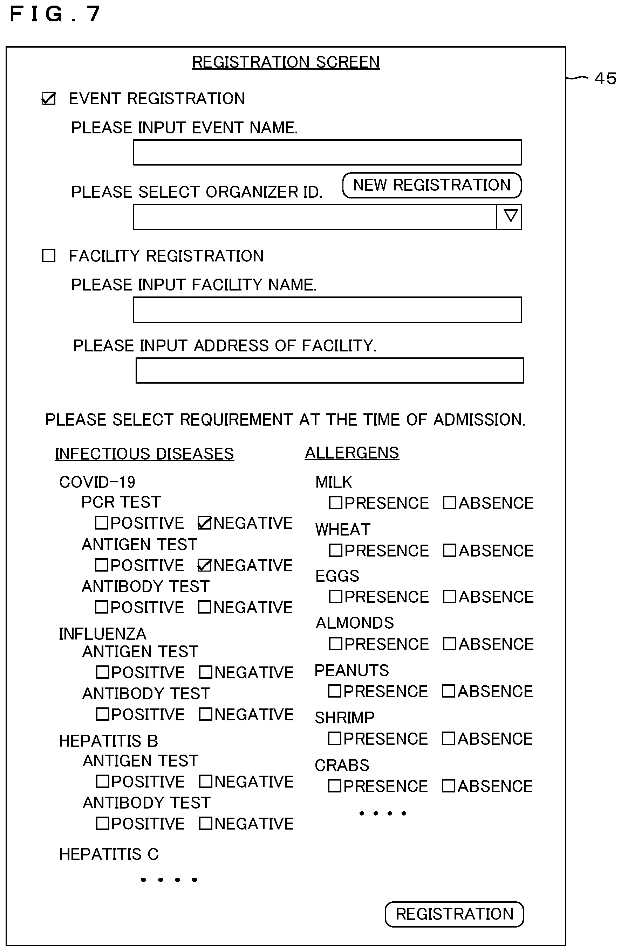

Processing is described by which the organizer of an event or the person in charge of a facility registers information on the event or the facility using the registration terminal 40. FIG. 6 is a flowchart of one example of a registration processing procedure while FIG. 7 is a schematic diagram illustrating a screen example. FIG. 6 shows the processing performed by the registration terminal 40 on the left and the processing performed by the server 10 on the right. The following processing is implemented by the control unit 41 according to a control program 42P stored in the storage unit 42 of the registration terminal 40 and implemented by the control unit 11 according to the control program 12P stored in the storage unit 12 of the server 10. Part of the following processing may be implemented with dedicated hardware circuitry.

In the information processing system 100 of the present embodiment, the organizer of an event registers on the server 10 information on the event on which attendance restrictions are imposed based on the health record of the user using the registration terminal 40. In addition, the person in charge of a facility registers on the server 10 information on the facility on which entry restrictions are imposed based on the health record of the user using the registration terminal 40. Registration of the information on an event or the information on a facility may be performed, for example, from a website published by the server 10 via a network N or by executing a predetermined application program (e.g., PHR application 22AP stored on the user terminal 20). The control unit 41 of the registration terminal 40 acquires a registration screen for registering event information or facility information from the server 10 and displays it on the display unit 45, in accordance with an operation by the user through the input unit 44 (S11).

FIG. 7 shows an example of the registration screen. The registration screen has checkboxes for selecting either event registration or facility registration, and an event name input field and an organizer ID input field that are allowed to be input when the event registration is selected and a facility name input field and an address input field that are allowed to be input when the facility registration is selected. The event name input field is configured to receive input of arbitrary text data. The organizer ID input filed includes a pull-down menu to select any one of the organizer IDs already registered in the server 10. It is noted that a new registration button to instruct the user to register a new organizer is provided near the organizer ID input field. The facility name input field and the address input field are configured to receive input of arbitrary text data. In addition, the registration screen includes input fields for a requirement (attendance requirement or entry requirement) at the time of admission when attendance restrictions or entry restrictions are imposed. The requirement input field has checkboxes to select any of the test results (positive or negative) for various infectious diseases and any of allergy test results (the presence or absence of allergy) for various allergens. Specifically, examples of the infectious diseases include COVID-19, influenza virus, hepatitis B virus, hepatitis C virus and the like. Example of the test items for each of the infectious diseases include a PCR test, an antigen test and an antibody test. Checkboxes are provided to select positive or negative for each of the test items. Examples of the allergens include milk, wheat, eggs, almonds, peanuts, shrimp and crabs. Checkboxes are provided to make a selection between allergic or not allergic to each of the allergens (test items). The requirements at the time of admission are not limited to the test results (positive or negative) of each of the test items for the infectious diseases or the test results (the presence or absence of allergy) for each of the allergens of the allergy test, and various test items may be input (selected). Checkboxes as to whether or not the user has been vaccinated, how many times the user has been vaccinated (e.g., one dose or two doses) and/or whether or not the user has been vaccinated within a predetermined period of time may be set as requirement input fields. The above-mentioned requirements may be conditional if any one of the requirements is satisfied, or if more than one requirements are satisfied. The registration screen has a registration button to instruct the user to register the input details. The configuration of the registration screen is not limited to that in FIG. 7.

In the case where the event organizer or the person in charge of the facility inputs the details to be registered in each of the input fields through the input unit 44 and instructs the registration of the input details on the registration screen illustrated in FIG. 7, he/she operates the registration button. The control unit 41 accepts registration details (event information or facility information) for each of the input fields on the registration screen (S12), and displays the accepted event information or facility information in each of the input fields. The control unit 41 determines whether or not the registration button on the registration screen is operated (S13). If determining that the registration button is not operated (S13: NO), the control unit 41 repeats the processing at step S12. If determining that the registration button is operated (S13: YES), the control unit 41 sends the event information or facility information received via the registration screen to the server 10, and requests the server 10 to register the event information or facility information (S14).

If receiving the registration request for new event information or facility information from the registration terminal 40, the control unit 11 of the server 10 issues an event ID or facility ID (S15) and stores the received event information or facility information (registration information) in the event DB 12b or facility DB 12c in association with the issued event ID or facility ID (S16). The control unit 11 then sends the issued event ID or facility ID to the registration terminal 40 (S17). If receiving the event ID or facility ID from the server 10, the control unit 41 of the registration terminal 40 displays the received event ID or facility ID on the display unit 45 (S18) to report the event ID or facility ID to the event organizer or the person in charge of the facility. It is noted that the control unit 41 may store the received event ID or facility ID in the storage unit 42. As a result of the processing described above, each information on the event or each information on the facility can be registered in the server 10. Thus, event information registered by the organizer for each of the events is registered in the event DB 12b of the server 10, while facility information registered by the person in charge of a facility for each of the facilities is registered in the facility DB 12c.

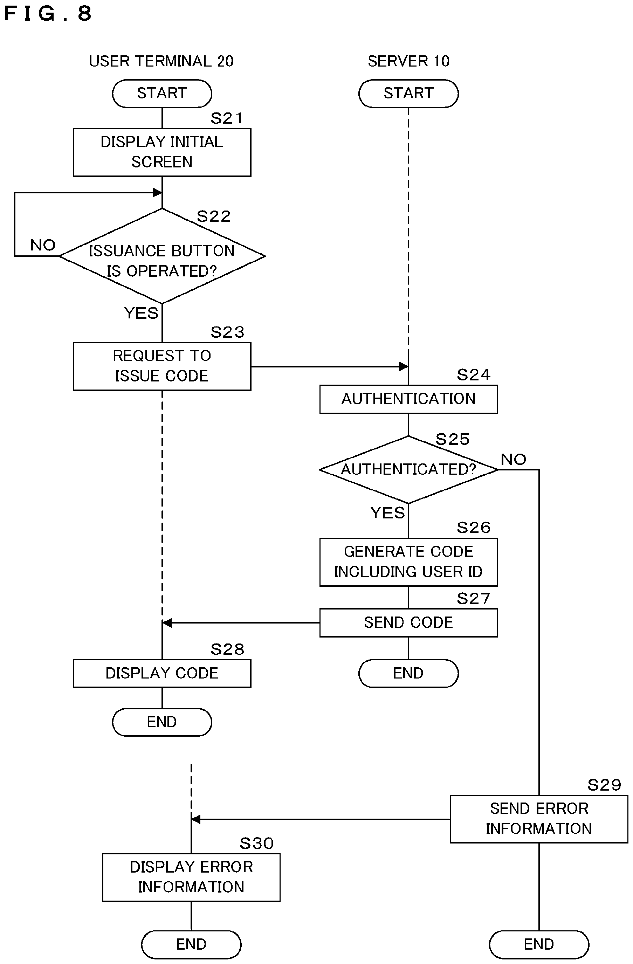

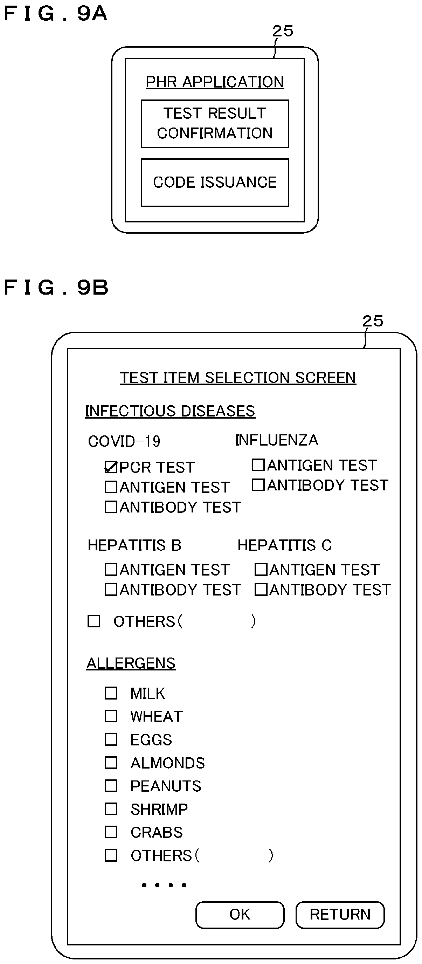

Next, processing is described by which a user who wishes to enter the facility (a user who wishes to attend an event) receives a code including user identification information from the server 10 using the user terminal 20. FIG. 8 is a flowchart of one example of the procedure of a code issuance processing while FIGs. 9A-9C are each a schematic diagram illustrating a screen example. FIG. 8 shows the processing performed by the registration terminal 20 on the left and the processing performed by the server 10 on the right. The following processing is implemented by the control unit 21 according to the control program 22P stored in the storage unit 22 of the user terminal 20, and implemented by the control unit 11 according to the control program 12P stored in the storage unit 12 of the server 10. Part of the following processing may be implemented with dedicated hardware circuitry.

In the information processing system 100 according to the present embodiment, before attending an event or before entering a facility, the user receives a code including the identification information of the user from the server 10 by activating the PHR application 22AP on the user terminal 20. If receiving an instruction to execute the PHR application 22AP through the input unit 24, the control unit 21 of the user terminal 20 activates the PHR application 22AP and displays the initial screen on the display unit 25 (S21). FIG. 9A illustrates an example of the initial screen. The initial screen has a test result confirmation button to instruct the user to confirm the test results having registered in the server 10 and a code issuance button to request the server 10 to issue a code. The PHR application 22AP can not only perform processing of receiving a code from the server 10 but also perform processing of receiving test results registered to the server 10. If the user operates the test result confirmation button on the initial screen, the control unit 21 displays a test item selection screen as illustrated in FIG. 9B on the display unit 25. If a test item is selected on the selection screen, the control unit 21 requests the server 10 to send a test result of the selected test item. If receiving the test result from the server 10, the control unit 21 displays it on the display unit 25. This allows the user to confirm his/her own test results registered in the server 10 using the user terminal 20. In the processing in FIG. 8, the confirmation processing of the test results is not described.

If wishing to receive a code from the server 10, the user operates the code issuance button on the initial screen. The control unit 21 of the user terminal 20 determines whether or not the code issuance button is operated (S22). If determining that the code issuance button is not operated (S22: NO), a standby state is held until the button is operated. If determining that the code issuance button is operated (S22: YES), the control unit 21 requests the server 10 to issue a code (S23). At this time, the control unit 21 sends the user ID of the user to the server 10 to request the issuance of a code.

If receiving the request to issue a code from the user terminal 20, the control unit 11 of the server 10 performs user authentication based on the user ID received from the user terminal 20 (S24). Here, the control unit 11 determines whether or not the received user ID is registered in the user DB 12a, and, if the user ID is registered, authenticates this user as a legitimate user. The control unit 11 determines whether or not the user is authenticated as a legitimate user (S25). If determining that the user is authenticated (S25: YES), the control unit 11 generates a code including the user ID received from the user terminal 20 (S26). In the present embodiment, the control unit 11 generates a QR code, but the code is not limited to the QR code.

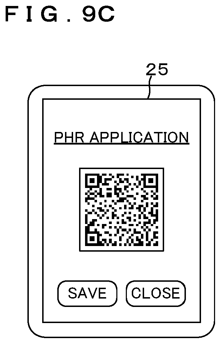

The control unit 11 then sends the generated code to the user terminal 20 (S27). If receiving the code from the server 10, the control unit 21 of the user terminal 20 displays the received code on the display unit 25 as illustrated in FIG. 9C (S28) and reports it to the user. The report screen shown in FIG. 9C has a save button to instruct the user to store the code that is being displayed in the PHR application 22AP and a close button to instruct the user to end the PHR application 22AP without storing the code. Thus, the control unit 21 stores the code that is being displayed in the PHR application 22AP if the save button on the report screen is operated, while the control unit 21 ends the display of the report screen if the close button is operated.

If determining that the user cannot be authenticated (S25: NO), the control unit 11 sends error information indicating that the user ID is not correct to the user terminal 20 (S29). If receiving the error information from the server 10, the control unit 21 of the user terminal 20 displays the received error information on the display unit 25 (S30) and reports it to the user. As a result of the above-mentioned processing, the legitimate user can receive the code including the identification information of the user (user ID) from the server 10.

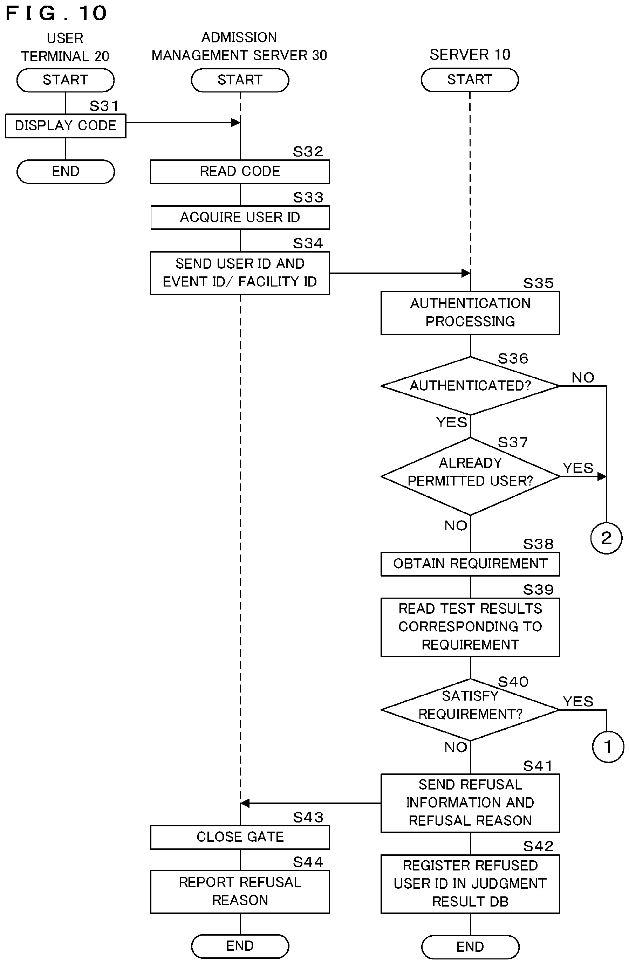

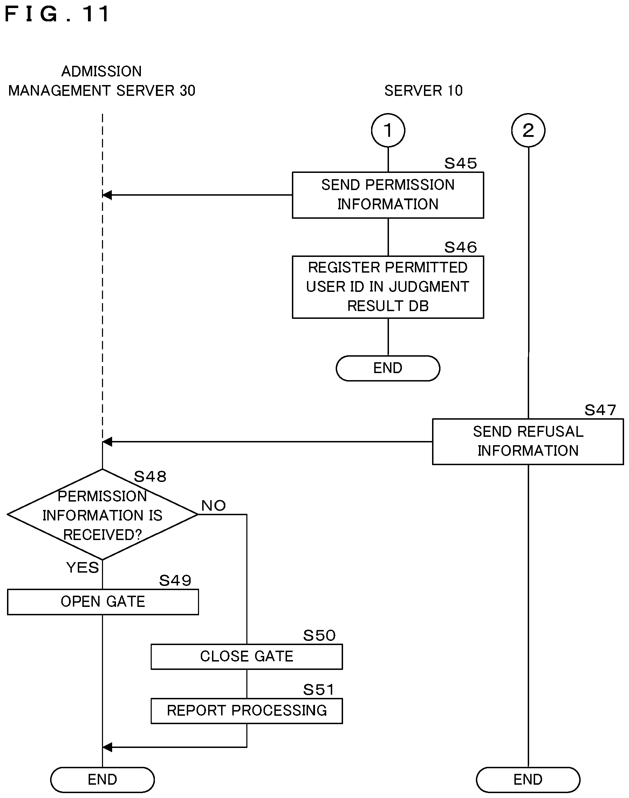

Next, processing is described by which judgment as to whether or not a user is permitted attendance or entry is performed based on the health record registered on the server 10 when the user attends an event or enters a facility. FIGs. 10 and 11 are each a flowchart of one example of a judgment processing procedure. FIG. 10 shows the processing performed by the user terminal 20 on the left, the processing performed by the admission management server 30 at the center, and the processing performed by the server 10 on the right. FIG. 11 shows the processing performed by the admission management server 30 on the left and the processing performed by the server 10 on the right. The following processing is implemented by the control unit 21 according to the control program 22P stored in the storage unit 22 of the user terminal 20, implemented by the control unit 31 according to the control program 32P stored in the storage unit 32 of the admission management server 30, and implemented by the control unit 11 according to the control program 12P stored in the storage unit 12 of the server 10. Part of the following processing may be implemented with dedicated hardware circuitry.

In the information processing system 100 according to the present embodiment, when attending an event or entering a facility, the user activates the PHR application 22AP to display the code received from the server 10 on the user terminal 20 to allow the gate device 30a to read the code as described above. The code from the server 10 that is received by the user terminal 20 may be employed by being printed on a recording paper or a card, for example. This allows even an elementary school student who does not have the user terminal 20 to utilize the information processing system 100 according to the present embodiment.

If receiving an instruction to execute the PHR application 22AP and to display the code through the input unit 24, the control unit 21 of the user terminal 20 activates the PHR application 22AP and displays the code saved in the PHR application 22AP on the display unit 25 (S31). At this time, the control unit 21 may display the screen as shown in FIG. 9C. By holding the user terminal 20 with the code displayed over the code reader 36 of the gate device 30a, the user causes the code reader 36 to read the code. The control unit 31 of the admission management server 30 reads the code with the code reader 36 (S32) and acquires the user ID included in the code (S33). This allows the control unit 31 (user information acquisition unit) to acquire the identification information of the user (user ID). The control unit 31 sends, to the server 10, the acquired user ID and the event ID of the event for which the admission management server 30 manages the attendance of the user, or the facility ID of the facility for which the admission management server 30 manages the entry of the user (S34) to request the server 10 to judge whether or not the user is permitted to attend the event or to enter the facility. In the processing shown in FIG. 6, the event ID or the facility ID has been received from the server 10 when the registration terminal 40 registers event information or facility information to the server 10, and stored in the storage unit 32 of the admission management server 30, for example.

When receiving a request to judge whether or not the user is permitted to attend the event or to enter the facility from the admission management server 30, the control unit 11 (acquisition unit) of the server 10 receives the user ID having read from the code by the admission management server 30 and performs the authentication processing based on the received user ID (S35). The control unit 11 here determines whether or not the received user ID is registered in the user DB 12a and determines that the user is a legitimate user if it is registered. The control unit 11 determines whether or not the user is authenticated as a legitimate user (S36) and determines whether or not the authenticated user is a user who has already been permitted to attend the event or enter the facility (S37) if determining that the user is authenticated (S36: YES). The control unit 11 here determines whether or not the user ID of the authenticated user has already been registered as a permitted user in the judgment result DB 12d in association with the event ID or facility ID received from the admission management server 30. If the user ID of the authenticated user is registered as a permitted user, the control unit 11 determines that the user is an already-permitted user.

If determining that the user is not a permitted user (S37: NO), the control unit 11 obtains an attendance requirement for this event or an entry requirement for this facility (S38). Specifically, if having received an event ID from the admission management server 30, the control unit 11 reads the attendance requirement corresponding to the event ID from the event DB 12b while if having received a facility ID, the control unit 11 reads the entry requirement corresponding to the facility ID from the facility DB 12c. The control unit 11 (readout unit) reads the test results of the authenticated user from the user DB 12a for the test items corresponding to the read attendance requirement or entry requirement (S39). For example, if reading the requirement "antigen test for COVID-19: Negative," the control unit 11 reads the result of the antigen test for COVID-19 of the authenticated user from the test result of the infectious disease stored in the user DB 12a. The control unit 11 then determines whether or not the read test result of the user satisfies the requirement obtained at step S38 (S40). For example, if the requirement is "antigen test for COVID-19: Negative," the control unit 11 determines that the requirement is satisfied if the read test result is negative, and that the requirement is not satisfied if the read test result is positive. If the requirement includes multiple test items, such as "antigen test for COVID-19: Negative and PCR test for COVID-19: Negative," the control unit 11 determines whether or not the test results satisfy the requirement for all the test items. In addition, the control unit 11 determines whether the test date of the read test result falls within the expiration period, and may determine that the requirement is not satisfied if the expiration period has elapsed.

If determining that the test result of the user does not satisfy the requirement obtained at step S38 (S40: NO), the control unit 11 (output unit) sends the refusal information (information on the test result) indicating that the user is refused attendance or entry and the reason for refusal to the admission management server 30 (S41). The reason for refusal includes information indicating the test item that fails to satisfy the attendance requirement or the entry requirement among the test results of the user. For example, the reason for refusal may include that the test result of the user is not the test result set to the attendance requirement or the entry requirement, that the user has not received the test for the test item set in the attendance requirement or the entry requirement, or that the expiration period has elapsed for the test result of the user. In addition, if multiple test items are included in the requirement, the reason for refusal may include the test items that satisfy the requirement and the test items that do not satisfy the requirement. The control unit 31 (health record acquisition unit) of the admission management server 30 receives the refusal information judged by the server 10. If receiving the refusal information from the server 10, the control unit 31 closes the gate 38a with the gate opening/closing unit 38 (S43) and reports the reason for refusal by performing report processing with the report unit 37 (S44). If the report unit 37 has a light output unit, the control unit 31 causes the light output unit to light or blink, for example, and if the report unit 37 has a sound output unit, it outputs sound, for example, to thereby report the reason for refusal. Moreover, the control unit 31 may make a report by displaying a report message on the display unit 35. This allows the admission management server 30 to report to the user passing through the entrance passage and the event organizer or the person in charge of the facility staying nearby that the user is refused to attend the event or enter the facility. The control unit 31 may perform different report processing depending on the details of the reason for refusal.

After processing at step S41, the control unit 11 of the server 10 registers the user ID of the user who is refused attendance or entry in association with the event ID or the facility ID in the refused user column of the judgment result DB 12d (S42). In addition, the number of refused users may be stored in the refused user column of the judgment result DB 12d. In this case, the control unit 11 adds one to the number of refused users stored in the judgment result DB 12d to make an update. If determining that the test result of the user satisfies the requirement obtained at step S38 (S40: YES), the control unit 11 (output unit) sends the permission information (information on the test result) indicating that the user is permitted attendance or entry to the admission management server 30 (S45). Then, the control unit 11 registers the user ID of the user who is permitted attendance or entry in association with the event ID or the facility ID in the permitted user column of the judgment result DB 12d (S46).

Meanwhile, if determining that the user is not authenticated at step S36 (S36: NO), or if determining that the user is an already-permitted user at step S37 (S37: YES), the control unit 11 sends the refusal information indicating that the user is refused attendance or entry to the admission management server 30 (S47). It is noted that the control unit 11 may send the reason for refusal of attendance or entry to the admission management server 30. Since the judgment processing to judge whether or not the attendance requirement or entry requirement is satisfied is not performed if the user cannot be authenticated, the target of the judgment processing can be limited to legitimate users. In addition, the refusal of the attendance or entry of the already-permitted user prevents overlapped issuance of the permission of attendance or the permission of entry based on the same user ID.

The control unit 31 (health record acquisition unit, judgement unit) of the admission management server 30 determines whether or not the permission information is received from the server 10 (S48). If determining that the permission information is received (S48: YES), the control unit 31 opens the gate 38 with the gate opening/closing unit 38 (S49). Here, the control unit 31 may perform report processing of reporting that attendance at the event or entry to the facility is permitted by the report unit 37. If determining that the permission information is not received (S48: NO), i.e., if the refusal information is received, the control unit 31 closes the gate 38a with the gate opening/closing unit 38 (S50) and performs report processing of reporting that attendance at the event or entry to the facility is refused by the report unit 37 (S51). Here, if having acquired the reason for refusal from the server 10, the control unit 31 may report the reason for refusal.

As a result of the above-mentioned processing, in the information processing system 100 according to the present embodiment, judgment is performed as to whether or not the attendance requirement or the entry requirement set for each event or each facility is satisfied based on the information of the test result of the user having registered on the server 10 when the user attends the event or enters the facility. Depending on whether or not the test result of the user satisfies the requirement, attendance at the event or entry to the facility is permitted or refused. Thus, the personal health record of each of the users having registered on the server 10 can be used for attendance restrictions or the entry restrictions imposed on the user by the admission management system, so that it is possible to effectively use the reliable test results conducted at a medical institution or the like. By thus imposing attendance restrictions or entry restrictions using the test results having registered on the server 10, the sense of security for the event or facility can be enhanced. This enables safe implementation of the event and safe use of the facility, which increases the reliability of the event and facility.

In the present embodiment, only the user ID is included in the code issued by the server 10, so that the code can also be used to attend a different event or to enter a different facility. Thus, by having saved the code issued by the server 10 in the PHR application 22AP of the user device 20, judgment as to whether or not the user is permitted attendance or entry can be performed by using the saved code in the case where the user attends a different event or enters a different facility. Moreover, since the user IDs of the users who are permitted attendance or entry are registered in the judgment result DB 12d in the present embodiment, the users who are permitted attendance or entry can be grasped for each event or facility. This makes it possible to grasp the number of users who attend each of the events and who enter each of the facilities. This can also grasp the users attended each event and entered each facility, so that the number of users attended may be used to, for example, charge the implementation of judgment processing for permission or refusal of attendance or entry.

In the present embodiment, the server 10 may issue a temporary ID at random when issuing a code in response to a request from the user terminal 20, issue a code including the temporary ID and the user ID, and provide the user terminal 20 with the code. Here, the admission management server 30 obtains the temporary ID and the user ID by reading the code displayed on the user terminal 20, and sends the acquired temporary ID and user ID as well as the event ID or facility ID to the server 10 to request the server 10 to judge whether or not the user is permitted attendance or entry. If the temporary ID received from the admission management server 30 matches the temporary ID stored in association with the user ID, the server 10 performs judgment processing as to whether or not the user is permitted attendance or entry. This configuration can prevent unauthorized use of the code. Moreover, the temporary ID may be configured to be discarded once authentication processing based on the temporary ID is performed, which can further prevent the unauthorized use of the code. In addition, an expiration period may be set to the code issued by the server 10, which also prevents the unauthorized use of the code.

In the present embodiment, when the user attends an event or enters a facility, the server 10 judges whether or not the user is permitted attendance or entry and reports the judgment result to the admission management server 30. In other words, since only the judgment result is sent from the server 10 to the admission management server 30, the test result (personal health record) of each of the users registered on the server 10 is prevented from leaking. It is noted that the admission management server 30 may be configured to judge whether or not the user is permitted attendance or entry. Here, the admission management server 30 obtains from the server 10 the test result used for judging whether or not attendance or entry is permitted, and judges whether or not the acquired test result satisfies the attendance requirement or entry requirement. In this case as well, attendance restrictions or entry restrictions can be imposed on the user by using the personal health record of each of the users having registered on the server 10. In such a configuration, the server 10 preferably sends the test result, which has been encoded, to the admission management server 30.

In the present embodiment, the event organizer or the person in charge of the facility has registered in advance in the server 10 the test items and test results to be used for the processing of judging whether or not the user is permitted attendance or entry as an attendance requirement or entry requirement, though it is not limited to such configuration. For example, the event organizer or the person in charge of the facility may report an attendance requirement or entry requirement to the user, and the user may report the attendance requirement or the entry requirement to the server 10 in advance when requesting the server 10 to issue a code. Alternatively, the admission management server 30 registers an attendance requirement or entry requirement in advance, and may send the attendance requirement or entry requirement to the server 10 when it requests the server 10 to judge whether or not the user is permitted attendance or entry. Even in such a configuration, the server 10 can judge whether or not the user is permitted attendance or entry based on the attendance requirement or entry requirement set for each event or each facility.

In the present embodiment, in addition to the test item and test result, a period (the term of validity) during which the test result is valid may be registered in the attendance requirement or entry requirement set for each event or each facility. The term of validity may, for example, be within three months from the date since the event was held. Here, when judging whether or not the test result of the user satisfies the attendance requirement or the entry requirement, the server 10 determines whether the test result is a test result obtained from the test conducted within the term of validity set to the attendance requirement or the entry requirement. This makes it possible to judge whether or not the user is permitted attendance or entry using the test result of the test conducted within the term of validity. It is noted that the server 10 may perform processing of periodically determining whether or not the term of validity for each test result has elapsed based on the test date of each test result stored in the user DB 12a, and successively deleting the expired test result from user DB 12a or changing the expiration flag from 0 to 1.

### Second Embodiment

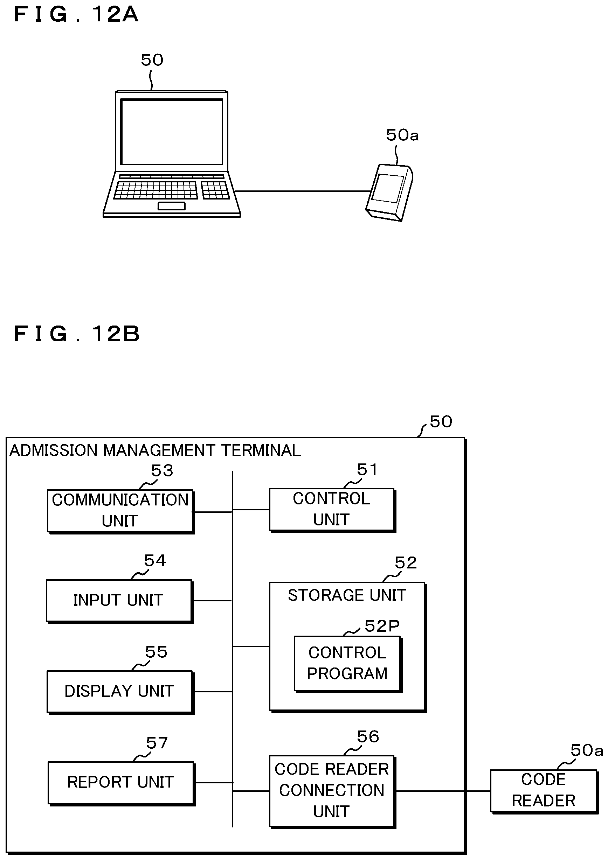

A modification of the admission management system will be described. FIG. 12A is a schematic diagram illustrating an admission management system according to a second embodiment. FIG. 12B is a block diagram illustrating an example of the configuration of the admission management system according to the second embodiment. In the information processing system 100 according to the present embodiment, the admission management system is composed of an admission management terminal 50 formed of an information processing device such as a personal computer and a tablet and a code reader 50a instead of the admission management server 30 and the gate device 30a. The rest of the configurations are similar to those of the first embodiment, and thus the concrete descriptions thereof will not be repeated.

The admission management terminal 50 and the code reader 50a may be configured to be connected through a cable or to make wireless communication. The admission management terminal 50 in the present embodiment includes a control unit 51, a storage unit 52, a communication unit 53, an input unit 54, a display unit 55, a code reader connection unit 56 and a report unit 57. The control unit 51, the storage unit 52, the communication unit 53, the input unit 54 and the display unit 55 of the admission management terminal 50 respectively have similar configurations to the control unit 11, the storage unit 12, the communication unit 13, the input unit 14 and the display unit 15 of the server 10 illustrated in FIG. 2, and the report unit 57 has a similar configuration to the report unit 37 of the gate device 30a illustrated in FIG. 5, and thus, the detailed descriptions thereof are not repeated. The code reader connection unit 56 is an interface for connecting to the code reader 50a and acquires information read by the code reader 50a separately provided from the admission management terminal 50.

In the admission management system thus configured, the code reader 50a is installed at a place where attendance restrictions or entry restrictions are imposed such as the entrance of a facility while the admission management terminal 50 is installed at any place or the like within the facility for use. It is noted that the display unit 55 or the report unit 57 may be configured to be separately provided from the admission management terminal 50. In this case, the display unit 55 or the report unit 57 may be installed at the entrance of the facility or the like together with the code reader 50a. The admission management system thus configured can be installed in a facility that does not have a gate at the entrance such as a shop and a small facility. The admission management system according to the present embodiment may have a gate to restrict the admission of the user to the facility. In this case, the admission management terminal 50 has a gate opening and closing unit. The gate opening and closing unit is configured to send an opening or closing instruction to the gate via wired or wireless communication.

In the information processing system 100 provided with the admission management system configured as described above also, each device executes processing similar to the ones in FIG. 6 and FIG. 8. Accordingly, in the present embodiment as well, the event information or facility information input via the registration terminal 40 is registered in the server 10, and a code requested via the user terminal 20 is issued by the server 10 and received by the user terminal 20. In the present embodiment as well, the devices each perform the same processing as shown in FIGs. 10 and 11. In the present embodiment, at steps S32 and S33 in FIG. 10, the control unit 51 of the admission management terminal 50 acquires through the code reader connection unit 56 the user ID obtained by the code reader 50a reading a code. The rest of the processing is similar to that of the first embodiment. If the information processing system 100 according to the present embodiment does not have the gate opening/closing unit 38 and the gate 38a, the control unit 31 of the admission management server 30 does not perform the processing at steps S43, S49 and S50 in FIGs. 10 and 11.

As a result of the above-mentioned processing, the present embodiment can also produce a similar effect to the first embodiment. Moreover, in the present embodiment, an admission management system is employed that is composed of the admission management terminal 50 formed of a personal computer, a tablet terminal or the like and the code reader 50a connected to the admission management terminal 50. Thus, even a facility that does not have a gate at the entrance can easily start the use of the information processing system 100 (personal health record managed by server 10).

### Third Embodiment

An information processing system will be described that performs user authentication based on authentication data other than the user ID when the server 10 judges whether or not the user is permitted attendance or entry. Since the information processing system 100 according to the present embodiment can be implemented with the same devices as the information processing system 100 of the first embodiment, the detailed description of the configuration will not be repeated. In the present embodiment, the user DB 12a, event DB 12b and facility DB 12c stored in the storage unit 12 of the server 10 are slightly different in configuration from those of the first embodiment as illustrated in FIG. 3 to FIG. 4B. In addition, the admission management server 30 is slightly different in configuration from that of the first embodiment illustrated in FIG. 5. Accordingly, only the differences are described.

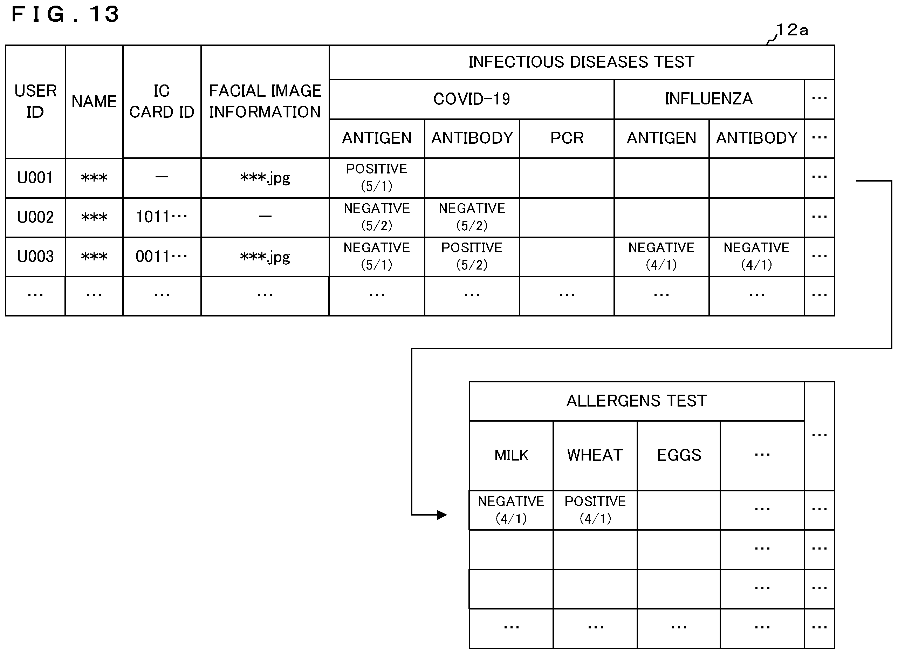

FIG. 13 is a schematic diagram illustrating an example of the configuration of the user DB 12a according to the third embodiment. The user DB 12a of the present embodiment further includes an IC (Integrated Circuit) card ID column and a facial image information column in the configuration according to the first embodiment illustrated in FIG. 3. The IC card ID column and the facial image information column respectively store the IC card ID and facial image information that are specified at the time of user registration in association with the user ID. The IC card ID is card information stored in the IC card assigned to the user, and the facial image information is image data obtained by photographing the face of the user. The IC card ID and facial image information are data used to authenticate the user and are not necessarily registered in the user DB 12a. In addition, the facial image information may not only be stored in the user DB 12a but also be stored in a predetermined area of the storage unit 12 or in another storage device. In this case, the facial image information column stores information (e.g., a file name indicating the storage location) for reading facial image information. The IC card ID and facial image information stored in the user DB 12a are stored by the control unit 11 if the control unit 11 obtains the respective information through the communication unit 13 or the input unit 14, and changed by the control unit 11 if the control unit 11 acquires a change instruction.

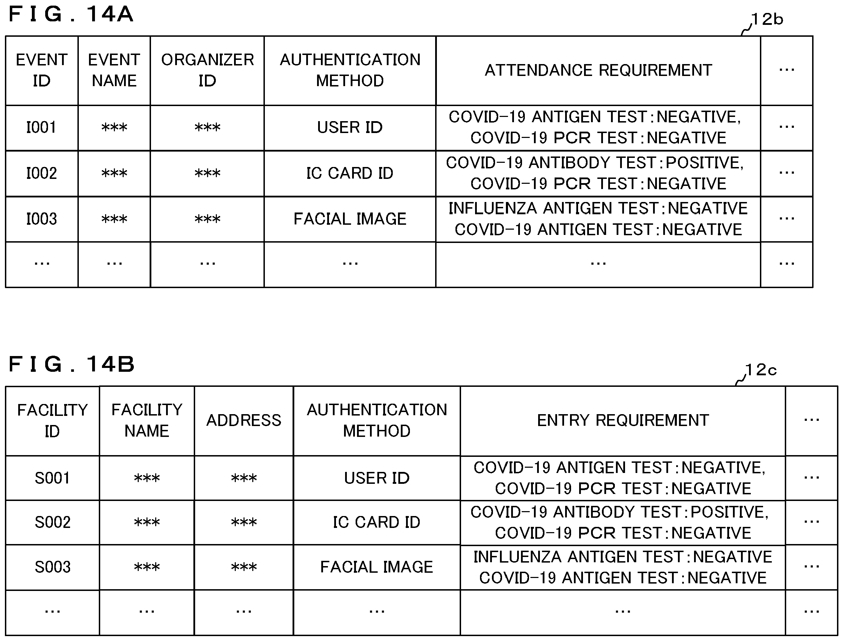

FIG. 14A is a schematic diagram illustrating an example of the configuration of the event DB 12b according to the third embodiment. FIG. 14B is a schematic diagram illustrating an example of the configuration of the facility DB 12c according to the third embodiment. The event DB 12b and facility DB 12c of the present embodiment each further include an authentication method column in the configuration of the first embodiment illustrated in FIGs. 4A and 4B. The authentication method column stores the type of data used to authenticate (identify) the user in association with the event ID or facility ID. It is noted that the authentication method is set for each event or for each facility together with the event information or facility information via the registration screen as shown in FIG. 7, for example, and employs a user ID, an IC card ID, a facial image and the like. The authentication method stored in the event DB 12b and the facility DB 12c is stored by the control unit 11 if the control unit 11 obtains it through the communication unit 13 or the input unit 14, and changed by the control unit 11 if the control unit 11 acquires a change instruction.

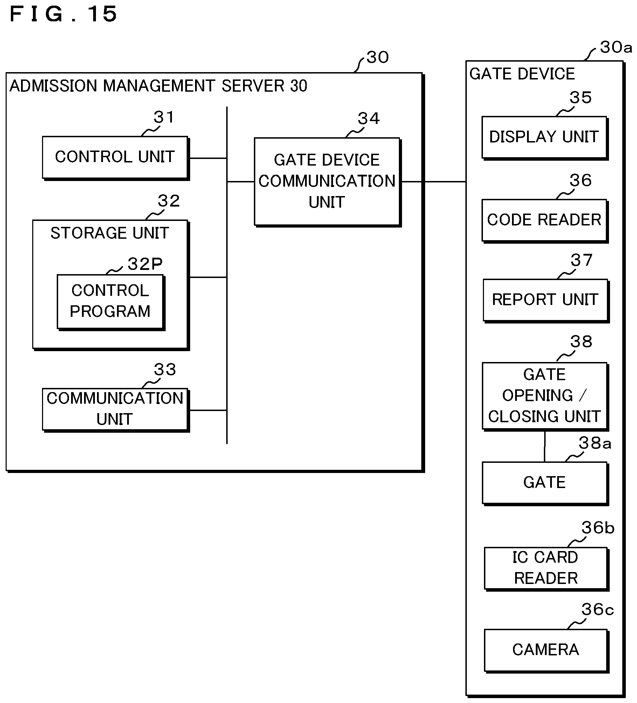

FIG. 15 is a block diagram illustrating an example of the configuration of an admission management system according to the third embodiment. The admission management system according to the present embodiment includes an IC card reader 36b and a camera 36c in addition to the configuration of the first embodiment illustrated in FIG. 5. The IC card reader 36b is a card reader that reads card information stored in an IC card. The camera 36c is an imaging device, performs photographing processing according to an instruction from the control unit 11, and sends the acquired image data (photographed images) to the storage unit 12 for storing the data. The IC card reader 36b and camera 36c may be configured not only to be integrated in the gate device 30a but also to be separately provided from the gate device 30a. In this case, the gate device 30a has a connection unit to which an external IC card reader or a camera can be connected, or has a wireless communication unit that can make wireless communication with an external IC card reader or a camera.

In the information processing system 100 according to the present embodiment, each device executes processing similar to the ones in FIG. 6 and FIG. 8. Accordingly, in the present embodiment as well, the event information or facility information input via the registration terminal 40 is registered in the server 10, and a code requested via the user terminal 20 is issued by the server 10 and received by the user terminal 20.

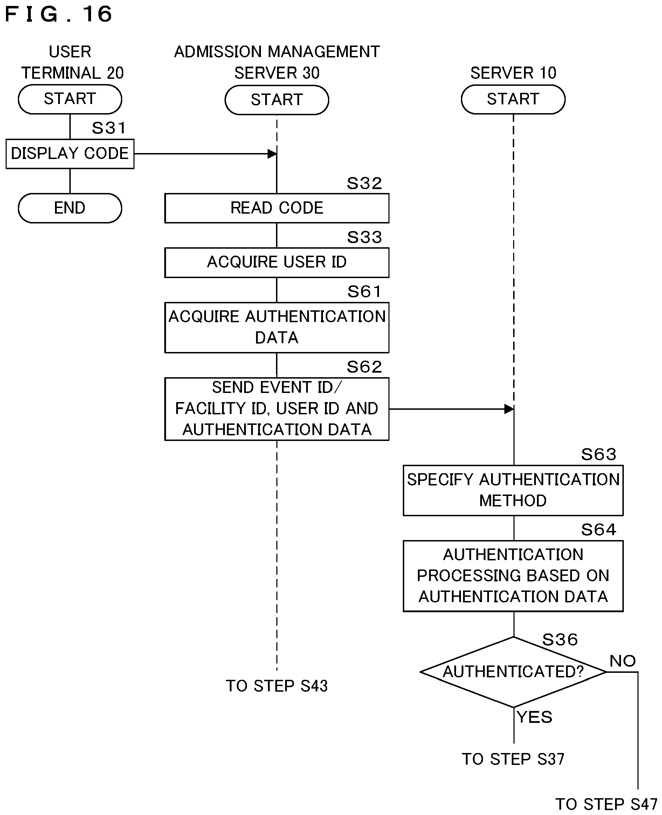

FIG. 16 is a flowchart of one example of a judgment processing procedure according to the third embodiment. The processing in FIG. 16 is obtained by adding steps S61-S62 in place of step S34 and by adding steps S63-S64 in place of step S35 in the processing in FIG. 10. The description as to the same steps as in FIGs. 10 and 11 are not repeated. FIG. 16 does not illustrate steps S37-S51 in FIGs. 10 and 11. The control unit 21 of the user terminal 20 and the control unit 31 of the admission management server 30 according to the present embodiment perform similar processing to steps S31-S33 illustrated in FIG. 10. Next, the control unit 31 of the admission management server 30 acquires the authentication data (authentication information) of the user using the IC card reader 36b or the camera 36c (S61). The authentication data used for user authentication is set for each event or each facility. By causing the gate device 30a to read the code, the user allows the gate device 30a to read the authentication data set for the event the user attends or the facility the user enters. Specifically, the control unit 31 reads the card ID stored in the IC card with the IC card reader 36b, or acquires the facial image of the user with the camera 36c.

The control unit 31 sends the event ID or facility ID and the acquired user ID and the acquired authentication data to the server 10 (S62) and requests the server 10 to judge whether or not the user is permitted to attend the event or enter the facility. When receiving a request from the admission management server 30 to judge whether or not the user is permitted to attend the event or enter the facility, the control unit 11 of the server 10 specifies an authentication method set to the event of the received event ID or the facility of the facility ID (S63). Specifically, if receiving the event ID, the control unit 11 reads an authentication method corresponding to the event ID from the event DB 12b while if receiving the facility ID, the control unit 11 reads an authentication method corresponding to the facility ID from the facility DB 12c. The control unit 11 then performs authentication processing based on the authentication data received from the admission management server 30 (S64) according to the read authentication method. If reading the authentication method by means of the user ID, for example, the control unit 11 authenticates the user depending on whether or not the received user ID is registered in the user DB 12a. Furthermore, if reading the authentication method by means of the IC card ID, the control unit 11 performs the authentication processing based on the received IC card ID (authentication data). The control unit 11 here determines whether or not the received IC card ID matches the IC card ID registered in association with the received user ID in the user DB 12a and determines that the user is a legitimate user if they match each other. Moreover, if reading the authentication method by means of a facial image, the control unit 11 determines whether or not the received facial image information matches the facial image information registered in association with the received user ID in the user DB 12a and determines that the user is a legitimate user if they match each other.

Then, the control unit 11 determines whether or not the user is authenticated as a legitimate user (S36), and shifts the processing to step S37 if the control unit 11 determines that the user is authenticated (S36: YES) and shifts the processing to step S47 if the control unit 11 determines that the user is not authenticated (S36: NO). As a result of the above-mentioned processing, in the information processing system 100 according to the present embodiment, if requesting the server 10 to judge whether or not the user is permitted attendance or entry, the admission management server 30 sends the authentication data using the authentication method depending on each event or each facility to the server 10. Thus, the server 10 can perform user authentication based on the authentication data other than the user ID when performing judgment as to whether or not the user is permitted attendance or entry, thereby enhancing security. It is noted that the authentication data is not limited to the IC card ID or the facial image information, but may be various biometric verification data such as fingerprints, palm veins, veins of the fingers of the hand, iris of the eye, voice prints or the like or may be a combination of multiple biometric information for authentication.

In the present embodiment, the effect similar to each of the embodiments described above can be obtained. In addition, in the present embodiment, in the case where the server 10 judges whether or not the user is permitted attendance or entry, the user authentication using not only the user ID but also the IC card ID or facial image is performed, which can enhance the security. In the present embodiment as well, the modification appropriately described in each of the above-mentioned embodiments is applicable. In addition, the configuration of the present embodiment can be applied to the information processing system 100 of the second embodiment, and can produce a similar effect even if it is applied to the information processing system 100 of the second embodiment. Specifically, the admission management system according to the second embodiment may be employed in the configuration of the present embodiment.

### Fourth Embodiment

An information processing system will be described that, when a user attends the event or enters the facility, acquires biometric information of the user in addition to the test results (health record) of the user registered on the server 10 and judges whether or not the user is permitted attendance or entry using the test results and the biometric information. Since the information processing system 100 according to the present embodiment can be implemented with the same devices as the information processing system 100 of the first embodiment, the detailed descriptions of their configurations will not be repeated. Since the gate device 30a of the present embodiment is slightly different in configuration from that of the first embodiment illustrated in FIG. 5, only the difference is described.

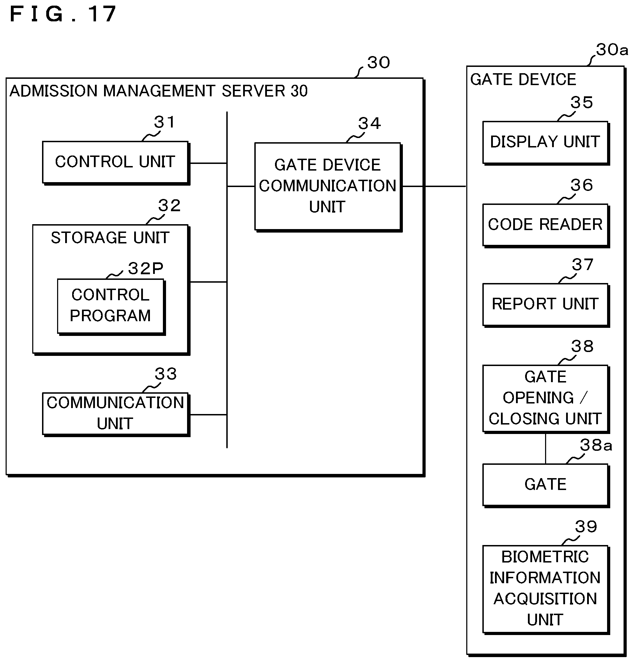

FIG. 17 is a block diagram illustrating an example of the configuration of an admission management system according to the fourth embodiment. In the admission management system according to the present embodiment, the gate device 30a includes a biometric information acquisition unit 39 in addition to the configuration of the first embodiment illustrated in FIG. 5. The biometric information acquisition unit 39 acquires biometric information such as body temperature, respiratory rate, heart rate and the like of the user passing through an entrance passage. The biometric information acquisition unit 39 has a sensor that detects biometric information of the user and acquires the biometric information detected by the sensor. The sensor may detect biometric information while in contact with the user, or may detect biometric information in a noncontact state. The biometric information acquisition unit 39 may be configured to acquire biometric information detected by a wearable device worn by the user.

In the information processing system 100 according to the present embodiment, each device executes processing similar to the ones in FIG. 6 and FIG. 8. Accordingly, in the present embodiment as well, the event information or facility information input via the registration terminal 40 is registered in the server 10, and a code requested via the user terminal 20 is issued by the server 10 and received by the user terminal 20.

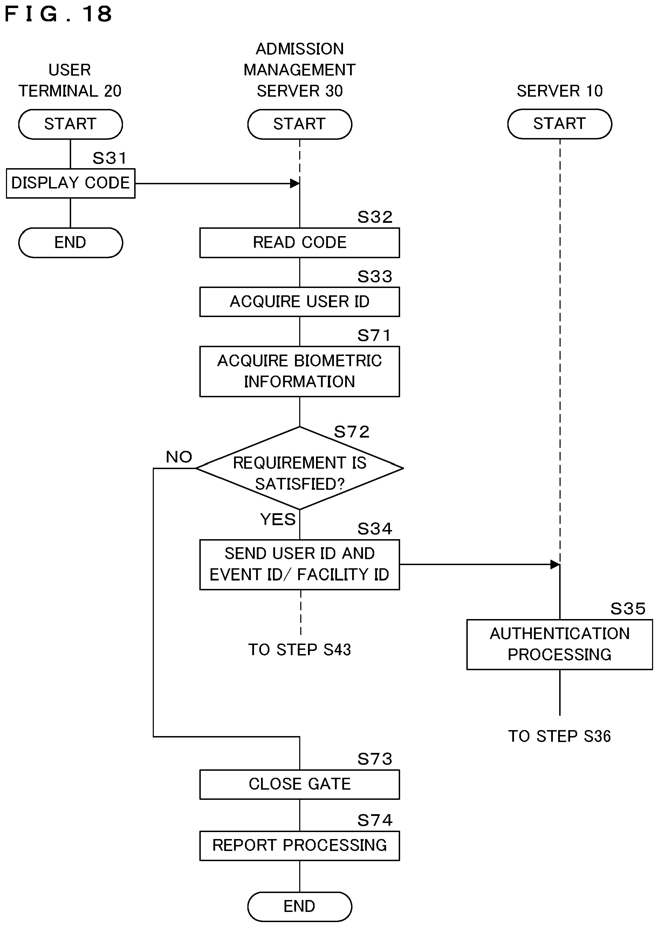

FIG. 18 is a flowchart of one example of a judgment processing procedure according to the fourth embodiment. The processing in FIG.18 is obtained by adding steps S71-S74 between steps S33 and S34 of the processing in FIG. 10. The description as to the same steps as in FIGs. 10 and 11 are not repeated. FIG. 18 does not illustrate steps S36-S51 in FIGs. 10 and 11. The control unit 21 of the user terminal 20 and the control unit 31 of the admission management server 30 according to the present embodiment perform similar processing to steps S31-S33 in FIG. 10. Next, the control unit 31 of the admission management server 30 acquires the biometric information of the user carrying the user terminal 20 (the user proceeding through the entrance passage) through the biometric information acquisition unit 39 (S71).

The control unit 31 determines whether or not an attendance requirement or an entry requirement is satisfied based on the acquired biometric information of the user (S72). The requirement here is that the body temperature, respiratory rate, heart rate and the like fall within a predetermined range and is stored in advance in the storage unit 32. Examples used as the attendance requirement or entry requirement include that the body temperature is lower than 37. 5 °C, that the respiratory rate is in the range of 10 to 20 breaths per minute, and that the heart rate is in the range of 50 to 100 beats per minute. Requirements for various tests that can be tested from exhaled breath such as wearing a mask and alcohol concentration in exhaled breath may also be used. Moreover, if the biometric information acquisition unit 39 is configured to acquire the age or gender of the user, the attendance requirement or the entry requirement may be registered for each age group or for each gender.

If determining that the biometric information of the user satisfies the requirement (S72: YES), the control unit 31 shifts the processing to step S34, and the admission management server 30 and the server 10 perform the processing at step S34 and after. If determining that the biometric information of the user does not satisfy the requirement (S72: NO), the control unit 31 closes the gate 38a with the gate opening/closing unit 38 (S73) and performs report processing with the report unit 37 (S74). Thus, the admission management server 30 can refuse the user attending an event or entering a facility if the biometric information of the user obtained when the user passing through the entrance passage does not satisfy the attendance requirement or the entry requirement and can report that the attendance or entry is refused to the user. Here, if refusal is reported based on the biometric information of the user when the user passes through the entrance passage, the user, event staff or the like may be notified of physical deconditioning of the user.

As a result of the processing described above, in the information processing system 100 according to the present embodiment, when the user attends an event or enters a facility, judgment is performed as to whether or not the user is permitted attendance or entry based on the biometric information obtained when the user passes though the entrance passage in addition to the test results of the user having registered on the server 10. Since there is a time lag between the date when the test was conducted and the date when the user passes through the entrance passage, the test results registered on the server 10 may not accurately indicate the condition of the user when he/she passes through the entrance passage. Thus, judgment as to whether or not the user is permitted attendance or entry is performed by a combined use of the test result conducted in advance with the biometric information obtained at the time of attendance at the event or entry to the facility, whereby more precise judgment is made possible. Thus, the attendance restrictions and entry restrictions can be imposed taking more precise judgment of the condition of the user into account.

In the present embodiment, the effect similar to each of the embodiments described above can be obtained. In addition, in the present embodiment, since judgment as to whether or not the user is permitted attendance or entry is performed based on the result of the tests conducted before the user attends the event or enters the facility and the biometric information obtained at the time when the user attends the event or enters the facility, more precise attendance restrictions and entry restrictions can be imposed. In the present embodiment as well, the modification appropriately described in each of the above-mentioned embodiments is applicable. In addition, the configuration of the present embodiment can be applied to the information processing system 100 according to the second to third embodiments, and can produce a similar effect even if it is applied to the information processing system 100 of the second to third embodiments.

### Fifth Embodiment

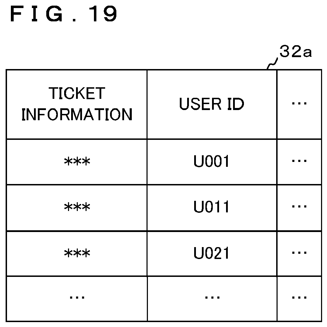

An information processing system including the gate device 30a located at the boarding gate of an airline will be described. Since the information processing system 100 according to the present embodiment can be implemented with the same devices as the information processing system 100 of the first embodiment, the detailed descriptions of their configurations will not be repeated. The admission management server 30 according to the present embodiment includes a ticket DB 32a in the storage unit 32 in addition to the configuration of the first embodiment illustrated in FIG. 5. FIG. 19 is a schematic diagram illustrating an example of the configuration of the ticket DB 32a. The ticket DB 32a is a conversion table storing, in association with the user ID of a user who purchases the boarding ticket, ticket information on a boarding ticket of an airplane issued by the airline. The ticket information can employ the data (e.g., boarding ID) included in a code described in a boarding ticket (boarding pass) of an airplane, for example. Information on the boarding ticket and information on the user who purchased the boarding ticket may be stored in the ticket DB 32a. The ticket DB 32a may be stored in another storage device connected to the admission management server 30, or may be stored in another storage device with which the admission management server 30 can communicate over a network N.

In the information processing system 100 according to the present embodiment, each device executes processing similar to the one in FIG. 6. In the present embodiment, facility information on airport facilities is input via the registration terminal 40 and registered to the server 10. Moreover, in the present embodiment, the gate device 30a installed at the boarding gate of the airline is configured to read a code (e.g., QR code) on the boarding ticket of the airplane issued by the airline. The code on the boarding ticket may be printed on a ticket sheet or displayed on the user terminal 20. The admission management server 30 according to the present embodiment stores in the ticket DB 32a the ticket information of the boarding ticket purchased by each of the users in association with the user ID registered in the server 10. Thus, the admission management server 30 can convert the ticket information obtained by reading the code on the boarding ticket to the corresponding user ID. Accordingly, in the present embodiment, the user does not need to acquire the code issued by the server 10 using the user terminal 20. In other words, in the present embodiment, the code on the boarding ticket for an airplane issued by the airline is used instead of the code issued by the server 10, which eliminates the respective devices having to perform the processing in FIG. 8.

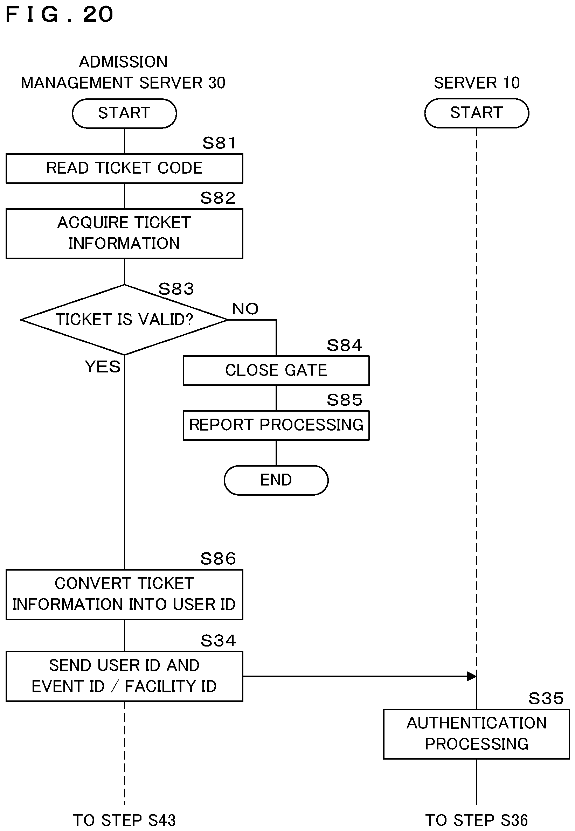

FIG. 20 is a flowchart of one example of a judgment processing procedure according to the fifth embodiment. The processing in FIG. 20 is obtained by adding steps S81-S86 in place of step S31-S33 of the processing in FIG. 10. The description as to the same steps as in FIGs. 10 and 11 are not repeated. FIG. 20 does not illustrate steps S36-S51 in FIGs. 10 and 11. The control unit 31 of the admission management server 30 according to the present embodiment reads the code on the boarding ticket issued by the airline with the code reader 36 (S81) and acquires the ticket information included in the code (S82).

The control unit 31 determines whether or not boarding an airplane is possible (whether or not the ticket is valid) based on the acquired ticket information (S83). For example, the ticket information on a valid ticket is stored in the storage unit 32 of the admission management server 30. The control unit 30 determines whether or not the ticket is valid depending on whether or not the ticket information read from the code is stored in the storage unit 32. If determining that the ticket is valid (S83: YES), the control unit 31 converts this ticket information into the user ID corresponding to the ticket information (S86). Specifically, the control unit 31 reads the user ID registered in the ticket DB32a in association with this ticket information. The control unit 31 then sends the converted user ID and the event ID or facility ID (the facility ID corresponding to an airline or a boarding gate at the airport, here) to the server 10 (S34), and requests the server 10 to judge whether or not the user is permitted entry (boarding the airplane). The control unit 11 of the server 10 performs the processing at and after step S35. Thus, the control unit 11 of the server 10 judges whether or not the user is permitted to board the airplane based on the user ID received from the admission management server 30, and sends the judgment result (permission information or refusal information) to the admission management server 30 that has read the ticket information to be converted.

If determining that the ticket is not valid (S83: NO), the control unit 31 closes the gate 38a with the gate opening/closing unit 38 (S84) and reports that boarding the airplane is refused by the report processing with the report unit 37 (S85). Here, the control unit 31 may report that boarding the airplane is impossible because the boarding ticket is not valid. As a result of the above-mentioned processing, the admission management server 30 according to the present embodiment can read the code on the boarding ticket for the airplane carried by the user to specify the user ID registered in the server 10 from the code on the boarding ticket. Since the user only needs to present the code on the boarding ticket at the time of boarding on an airplane, management of the code is made easy.

In the present embodiment, the processing of converting the ticket information to the user ID corresponding to the ticket information is not necessarily performed by the admission management server 30. Assuming that the ticket DB 32a is stored in a different device, for example, the control unit 31 of the admission management server 30 is configured to send the ticket information read by the code reader 36 to this different device and receive the user ID having converted by the different device. Moreover, the server 10 has stored the ticket DB 32a, and may perform the processing of converting ticket information into a user ID. In this case, the admission management server 30 sends the ticket information read by the code reader 36 to the server 10 while the server 10 may convert the received ticket information into a user ID based on the ticket DB 32a and perform judgment processing as to whether or not the user is permitted attendance or entry based on the convert user ID.

In the present embodiment, the effect similar to each of the embodiments described above can be obtained. Additionally, the present embodiment allows the server 10 to judge whether or not the user is permitted to board the airplane with only the code on the boarding ticket for the airplane, which improves user convenience. In the present embodiment as well, the modification appropriately described in each of the above-mentioned embodiments is applicable. In addition, the configuration of the present embodiment can also be applied to the information processing system 100 of the second to fourth embodiments, and can produce a similar effect even if it is applied to the information processing system 100 of the second to fourth embodiments.

### Sixth Embodiment

An information processing system will be described that generates, when the server 10 issues a code in response to a request from the user terminal 20, the code including the information on the ticket necessary to attend an event or enter a facility in addition to the identification information of the user. Since the information processing system 100 according to the present embodiment can be implemented with the same devices as the information processing system 100 of the first embodiment, the detailed descriptions of their configurations will not be repeated. In the information processing system 100 according to the present embodiment, the devices each perform the same processing as shown in FIG. 6. Thus, in the present embodiment as well, the event information or the facility information having input via the registration terminal 40 is registered in the server 10.

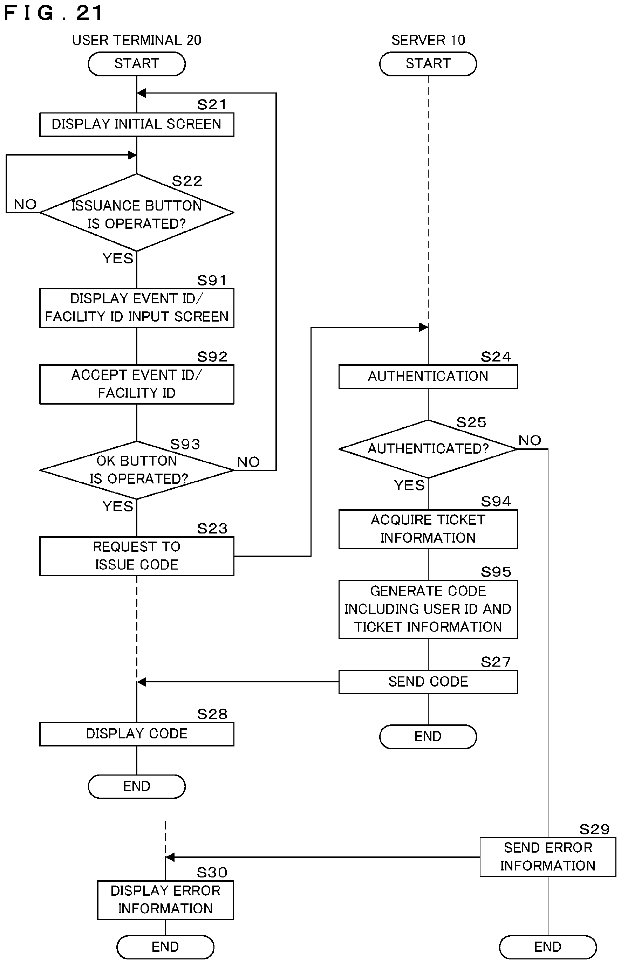

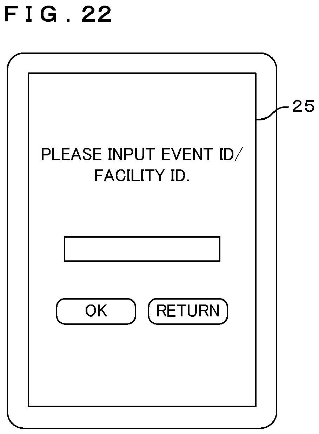

FIG. 21 is a flowchart of one example of the procedure of code issuance processing according to the sixth embodiment, while FIG. 22 is a schematic diagram illustrating a screen example. The processing in FIG. 21 is obtained by adding steps S91-S93 between steps S22 and S23 and by adding steps S94-S95 in place of step S26 in the processing in FIG. 8. The description as to the same steps as those in FIG 8 are not repeated. The control unit 21 of the user terminal 20 according to the present embodiment performs similar processing to steps S21-S22 illustrated in FIG. 8. In the present embodiment, if determining that the code issuance button is operated on the initial screen (S22: YES), the control unit 21 displays an input screen for inputting the event ID of the event which the user attends or the facility ID of the facility which the user enters on the display unit 25 (S91). FIG. 22 shows an example of the input screen. The input screen has an input field for an event ID or facility ID, an OK button to instruct the server 10 to issue a code based on the input event ID or facility ID, and a return button to return to the display of the initial screen. The user inputs the event ID of an event he/she wishes to attend or the facility ID of a facility he/she wishes to enter to the input field and then operates the OK button. The event ID or facility ID is an ID issued from the server 10 when the event information or facility information is registered on the server 10. The user has previously obtained the ID from the organizer of the event or the person in charge of the facility, for example.

The control unit 21 accepts an input of the event ID or facility ID performed on the input field of the input screen through the input unit 24 (S92), and displays the accepted event ID or facility ID in the input field. The control unit 21 then determines whether or not the OK button on the input screen is operated (S93). If determining that the OK button is operated (S93: YES), the control unit 21 shifts the processing to step S23 to request the server 10 to issue a code (S23). In the present embodiment, the control unit 21 sends the user ID of the user and the even ID or facility ID acquired at step S92 to the server 10 to request for the issuance of a code. If determining that the OK button is not operated (S93: NO), i.e., if the return button on the input screen is operated, the control 21 will return the processing to step S21. Specifically, the control unit 21 ends the display of the input screen and displays the initial screen again.

If determining that the user is authenticated (S25: YES), the control unit 11 of the server 10 acquires the ticket information of the ticket required to attend this event or to enter the facility based on the event ID or facility ID received from the user terminal 20 (S94). At a time of registration of the event information or facility information, for example, the server 10 has previously received the ticket information from the registration terminal 40 and registered it in the event DB 12b or facility DB 12c. The control unit 11 then generates a code including the user ID received from the user terminal 20 and the ticket information acquired at step S94 (S95), and sends the generated code to the user terminal 20 (S27). As a result of the above-mentioned processing, a code requested via the user terminal 20 is issued by the server 10 and received by the user terminal 20 in the present embodiment as well. In the present embodiment, the code provided by the server 10 is not only the identification information of the user (user ID), but also the ticket information required when the user attends the event or the ticket information required when the user enters the facility. This allows the user to present the user ID and the ticket information with a single code, which facilitates the management of a code.

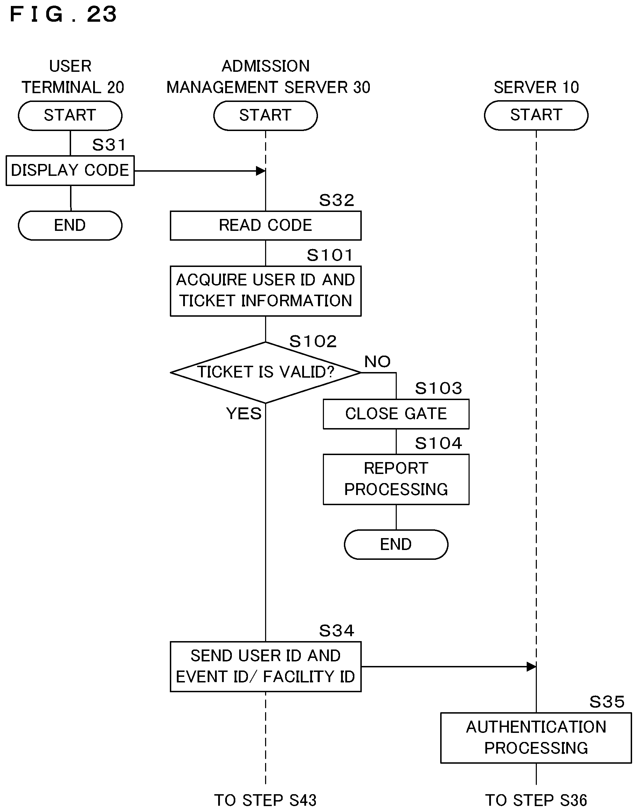

FIG. 23 is a flowchart of one example of a judgment processing procedure according to the sixth embodiment. The processing in FIG. 23 is obtained by adding steps S101-S104 in place of step S33 of the processing in FIG. 10. The description as to the same steps as those in FIGs. 10 and 11 are not repeated. FIG. 23 does not illustrate at steps S36-S51 of the processing in FIGs. 10 and 11. The control unit 21 of the user terminal 20 and the control unit 31 of the admission management server 30 according to the present embodiment perform similar processing to those at steps S31-S32 illustrated in FIG. 10. If reading the code, the control unit 31 of the admission management server 30 according to the present embodiment acquires the user ID and the ticket information included in the code (S101).

The control unit 31 determines whether or not attendance at the event or entry to the facility is possible (whether or not the ticket is valid) based on the acquired ticket information (S102). If determining that the ticket is valid (S102: YES), the control 31 shifts the processing to step S34 to send the user ID and the event ID or facility ID that are read from the code to the server 10 (S34) and requests the server 10 to judge whether or not the user is permitted attendance or entry.

If determining that the ticket is not valid (S102:NO), the control unit 31 closes the gate 38a with the gate opening/closing unit 38 (S103) and performs report processing with the report unit 37 (S104). As a result of the above-mentioned processing, in the present embodiment, the admission management server 30 can acquire the user ID and the ticket information that are used to request the server 10 to judge whether or not the user is permitted attendance or entry by reading the code displayed on the user terminal 20. The user only needs to present a single code when attending an event or entering a facility, which facilitates management of the code.

In the present embodiment, the code including the user ID and ticket information may be configured to not only be issued from the server 10e but also issued from the terminal on the event organizer side of the event or the terminal on the ticket issuer side. In this case, the terminal that issues the code may be configured to obtain the user ID of each user from the server 10, generate a code including the acquired user ID and ticket information of each user, and provide it to the user terminal 20 of each user.

In the present embodiment, the effect similar to each of the embodiments described above can be obtained. In the present embodiment, the user ID and the ticket information can be presented with a single code, which improves the convenience of the user. In the present embodiment as well, the modification appropriately described in each of the above-mentioned embodiments is applicable. In addition, the configuration of the present embodiment can be applied to the information processing system 100 of the second to fifth embodiments, and can produce a similar effect even if it is applied to the information processing system 100 of the second to fifth embodiments.

### Seventh Embodiment

An information processing system will be described that generates, when the server 10 issues a code in response to a request from the user terminal 20, a code including the information on the test result of the user in addition to the identification information (user ID) of the user. Since the information processing system 100 according to the present embodiment can be implemented with the same devices as the information processing system 100 of the first embodiment, the detailed descriptions of their configurations will not be repeated. In the information processing system 100 according to the present embodiment, the devices each perform the same processing as shown in FIG. 6. This allows the server 10 to register event information or facility information having been input via the registration terminal 40, in the present embodiment as well.

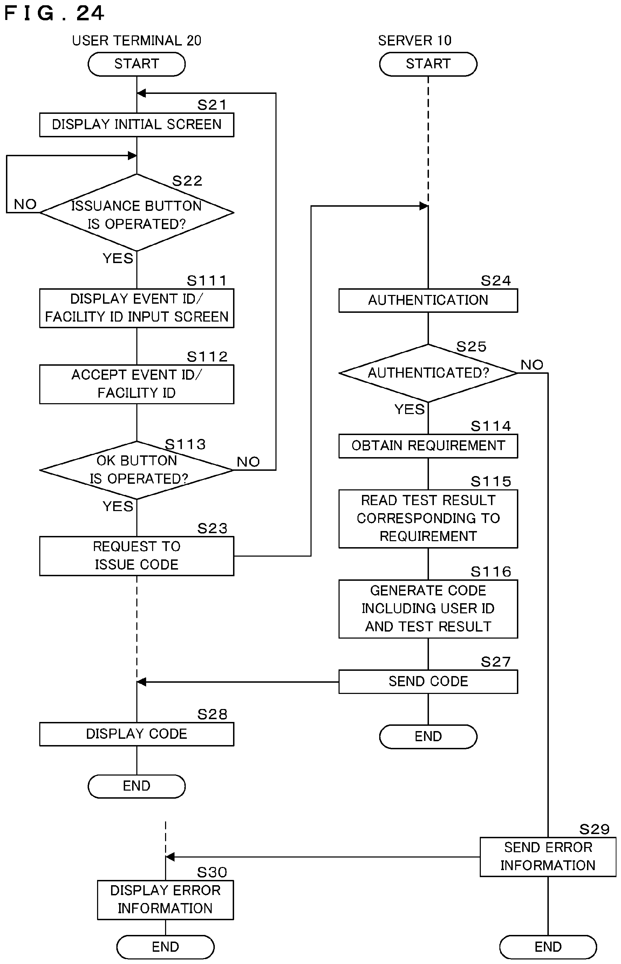

FIG. 24 is a flowchart of one example of the procedure of code issuance processing according to a seventh embodiment. The processing in FIG. 24 is obtained by adding steps S111-S113 between steps S22 and S23 and by adding steps S114-S116 in place of step S26 in the processing in FIG. 8. The description as to the same steps as those in FIG. 8 are not repeated. The control unit 21 of the user terminal 20 according to the present embodiment performs similar processing to steps S21-S22 and S91-S93 in FIG. 21 as in the sixth embodiment (S21-S22 and S111-S113). Thus, even in the present embodiment as well, the user terminal 20 sends the user ID and the event ID or facility ID acquired through the input screen to the server 10 to request issuance of a code.

If determining that the user is authenticated at step S25 (S25: YES), the control unit 11 of the server 10 obtains an attendance requirement set for this event from the event DB 12b or an entry requirement set for this facility from the facility DB 12c based on the event ID or facility ID received from the user terminal 20 (S114). The control unit 11 then reads from the user DB 12a the test result of the user with the received user ID for the test items corresponding to the read attendance requirement or entry requirement (S115). The control unit 11 then generates a code including the user ID and the test result of the user read at step S115 (S116), and sends the generated code to the user terminal 20 (S27).

As a result of the above-mentioned processing, a code requested via the user terminal 20 is issued by the server 10 and received by the user terminal 20 in the present embodiment as well. In the present embodiment, the code provided by the server 10 includes not only the user ID, but also the test results used for judging whether or not the user is permitted to attend the event or enter the facility. Thus, the user can acquire from the server 10 a code including test results for test items corresponding to the attendance requirement set for the event the user wishes to attend or the entry requirement set for the facility he/she wishes to enter.

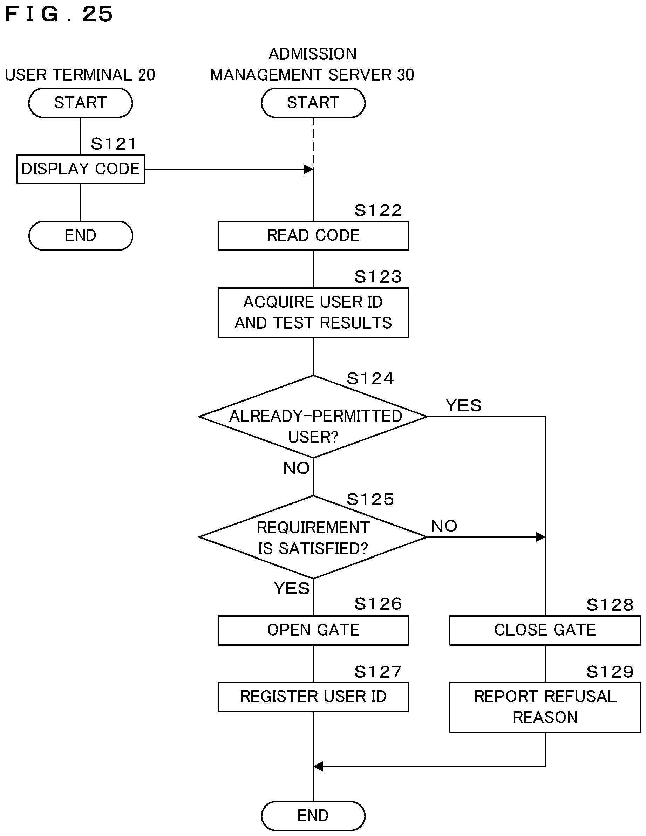

FIG. 25 is a flowchart illustrating one example of a judgment processing procedure according to the seventh embodiment. FIG. 25 shows the processing performed by the user terminal 20 on the left and the processing performed by the admission management server 30 on the right. The following processing is implemented by the control unit 21 according to the control program 22P stored in the storage unit 22 of the user terminal 20, and by the control unit 31 according to the control program 32P stored in the storage unit 32 of the admission management server 30. Part of the following processing may be implemented with dedicated hardware circuitry.

In the information processing system 100 according to the present embodiment as well, if receiving an instruction to execute the PHR application 22AP and to display a code, the control unit 21 of the user terminal 20 activates the PHR application 22AP and displays a code on the display unit 25 (S121). The user then causes the code reader 36 of the gate device 30a to read the code displayed on the user terminal 20. The control unit 31 of the admission management server 30 reads the code with the code reader 36 (S122) and acquires the user ID and the test results included in the code (S123). Here, the control unit 31 may be configured to acquire the user ID included in the code, request the server 10 to perform user authentication based on the user ID and perform the processing at and after S124 if the user is authenticated.

The control unit 31 then determines whether or not the user ID read from the code has already been registered (stored) as a user ID of the already-permitted user in the storage unit 32 (S124). In the present embodiment, since the control unit 31 has already registered the user ID of the user who has already been permitted attendance or entry in the storage unit 32 (DB not illustrated), whether or not the user is an already-permitted user can be determined depending on whether or not the user ID is registered in the storage unit 32. Alternatively, it may be possible that the control unit 31 sends the event ID or facility ID and the user ID to the server 10, and the server 10 may determine whether or not the user ID has already been registered as the user ID of the already-permitted user in the judgment result DB 12d stored in the server 10.

If determining that the user ID is not the user ID of the already-permitted user (S124: NO), the control unit 31 determines whether or not the test result read from the code satisfies the attendance requirement or the entry requirement set for the event or the facility that the admission management server 30 manages its attendance or entry (S125). It is noted that the attendance requirement or the entry requirement is stored in advance in the storage unit 32 of the admission management server 30. If determining that the test result of the user read from the code satisfies the attendance requirement or the entry requirement (S125: YES), the control unit 31 opens the gate 38a with the gate opening/closing unit 38 (S126). The control unit 31 then registers the user ID of the user whose attendance or entry is permitted as the user ID of the already-permitted user (S127).

If determining that the user is an already-permitted user (S124: YES), or if determining that the test result does not satisfy the requirement (S125: NO), the control unit 31 closes the gate 38a with the gate opening/closing unit 38 (S128) and reports the reason for refusal of attendance or entry by performing report processing with the report unit 37 (S129). If determining that the user is an already-permitted user, the control unit 31 reports that the user has already entered, while if determining that the test result does not satisfy the requirement, the control unit 31 reports the reasons why the attendance requirement or the entry requirement is not satisfied. For example, the control unit 31 reports that the test result of the user is different from the test result set for the requirement, that the user has not received the test for the test item set for the requirement, and that the expiration period of the test result of the user has elapsed. As a result of the above-mentioned processing, in the present embodiment, the admission management server 30 can obtain the test result of the user by reading the code displayed on the user terminal 20 and can judge whether or not the user is permitted attendance or entry based on the obtained test result. In this case as well, whether or not the user is permitted to attend the event or enter the facility can be judged based on the information of the test result of the user registered on the server 10.

In the present embodiment, the requirement for attendance at the event or the requirement for entry to the facility are not necessarily registered in advance on the server 10 (the event DB 12b or facility DB 12c). For example, when the organizer of the event or the person in charge of the facility reports an attendance requirement or entry requirement to the user, the user may report the attendance requirement or entry requirement to the server 10 when requesting the server 10 to issue a code. In such a configuration as well, the server 10 may generate a code including the test result of the user depending on the attendance requirement or entry requirement set for each event or each facility and provide it to the user terminal 20.

In the present embodiment, the effect similar to each of the embodiments described above can be obtained. In addition, in the present embodiment, since the judgment as to whether or not the user is permitted to attend an event or enter a facility is performed by the admission management server 30 alone, this eliminates the admission management server 30 having to communicate with the server 10, which can reduce the processing time. In the present embodiment as well, the modification appropriately described in each of the above-mentioned embodiments is applicable. In addition, the configuration of the present embodiment can also be applied to the information processing system 100 of the second to sixth embodiments, and can produce a similar effect even if it is applied to the information processing system 100 of the second to sixth embodiments. It is noted that in the case where the configuration of the present embodiment is applied to the information processing system 100 of the sixth embodiment, the server 10 generates a code including information on the test result of the user, the user ID and the ticket information and provides the user terminal 20 with the code. In addition, in the case where the configuration of the present embodiment is applied to the information processing system 100 of the fourth embodiment, attendance restrictions or entry restrictions can be imposed on the user based on not only the test result included in the code but also the biometric information at the time of attendance at the event or entry to the facility.

In the present embodiment, the information processing system configured to generate, but not limited to, the code including the information on the test result of the user in addition to the identification information of the user (user ID) is described. The information processing system may be configured to generate a code including vaccination history information in place of or in addition to the information on the test result of the user. The procedure of issuing a code can employ similar processing to those described with reference to FIG. 8 and FIGs. 9A-9C. In this case, the screen in FIG. 9A may be configured to display a vaccination history confirmation button, and the screen in FIG. 9B may be configured to display a list of multiple types of vaccines so as to a specific type of vaccine selectable and to issue a code. The screen in Fig. 9C may be configured to display a variety of information including a vaccination history using text information, optionally, test results of infectious diseases, and the name and the date of birth of a user other than the code. At this time, the code includes not only the user ID, but also information on the vaccination history specified by the user and optionally, the test result of the infectious diseases. By causing the admission management server 30 having stored a passage requirement in advance to read this code, it becomes possible to smoothly pass through a passing point. In addition, since the vaccination history information is displayed in text on the display screen, this allows an observer to visually check the passage requirement at the passing point.

### Eighth Embodiment

An information processing system will be described in which the admission management server 30 imposes entry restrictions based on the test result of the user registered on the server 10 and collects information on the allergic disease of the user when a user enters the facility of a store such as a restaurant or the like. It is noted that the information processing system according to the present embodiment may be configured to only perform processing of collecting information on the allergic disease of the user when the user enters the store. Since the information processing system 100 according to the present embodiment can be implemented with the same devices as the information processing system 100 of the first embodiment, the detailed descriptions of their configurations will not be repeated. The admission management server 30 according to the present embodiment may employ the admission management system according to the second embodiment illustrated in FIGs. 12A and 12B, and the code reader 36 may be installed at the entrance of the store, for example. The facility DB 12c stored in the storage unit 12 of the server 10 of the present embodiment is slightly different in configuration from the first embodiment illustrated in FIG. 4B.

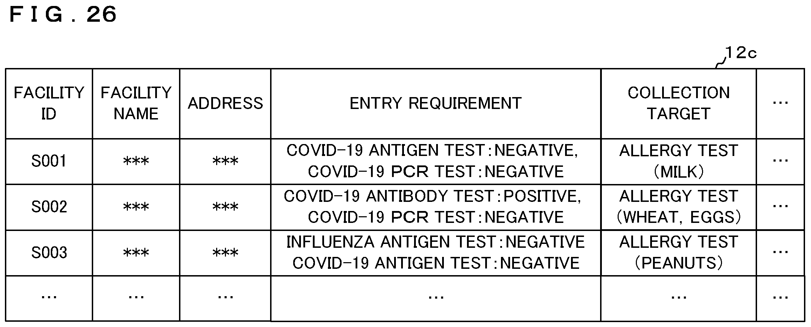

FIG. 26 is a schematic diagram illustrating an example of the configuration of the facility DB 12c according to an eighth embodiment. The facility DB12c of the present embodiment additionally includes a collection target column in the configuration of the first embodiment illustrated in FIG. 4B. The collection target column stores the test items for an allergy test (allergens) to be collected when each of the users enters the facility (the store). The items as collection targets are stored by the control unit 11 if the control unit 11 obtains the information through the communication unit 13 or the input unit 14, and changed by the control unit 11 if a change instruction is acquired. It is noted that the event DB 12b of the present embodiment may also be configured to include the collection target column.

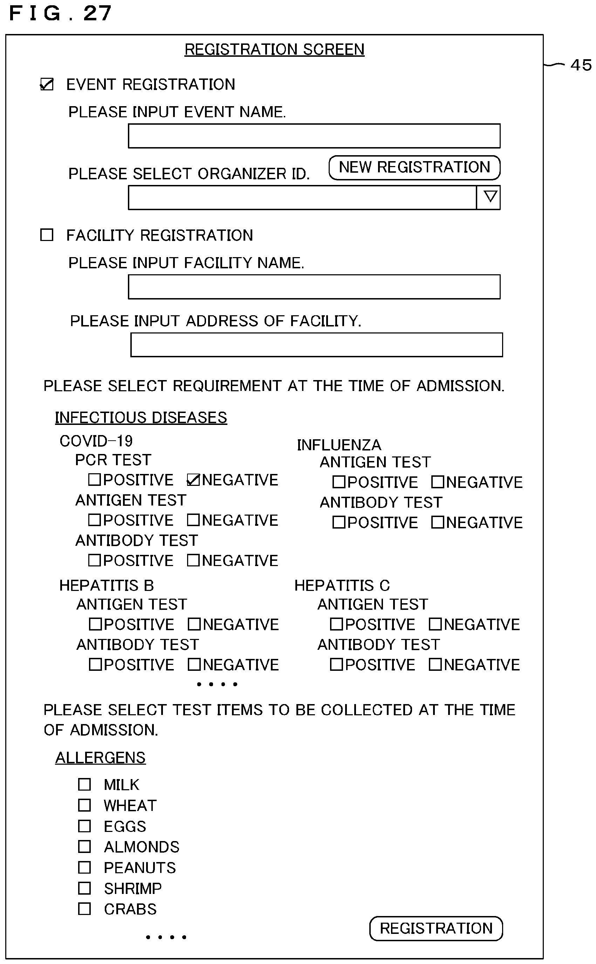

In the information processing system 100 according to the present embodiment, each device executes processing similar to the one in FIG. 6. Accordingly, in the present embodiment as well, the event information or facility information input via the registration terminal 40 is registered in the server 10. In the present embodiment, the registration terminal 40 accepts event information or facility information through a registration screen as illustrated in FIG. 27 and sends the accepted event information or facility information to the server 10, and the server 10 registers the information. FIG. 27 is a schematic diagram illustrating an example of the registration screen. The registration screen illustrated in FIG. 27 has a configuration similar to the registration screen shown in FIG. 7, and additionally includes input fields for test items for which test results are to be collected when the user enters a facility (store). On the screen illustrated in FIG. 27, checkboxes for selecting the test result for each of the test items of the infectious disease are provided as a requirement for imposing entry restrictions, and checkboxes for selecting any of the allergens for the allergic test (test items) are provided as test items for which the test results are to be collected when the user enters the facility. However, the registration screen is not limited to such a configuration.

In the information processing system 100 according to the present embodiment, each device executes processing similar to the one in FIG. 8. As a result of the above-mentioned processing, a code requested via the user terminal 20 is issued by the server 10 and received by the user terminal 20 in the present embodiment as well.

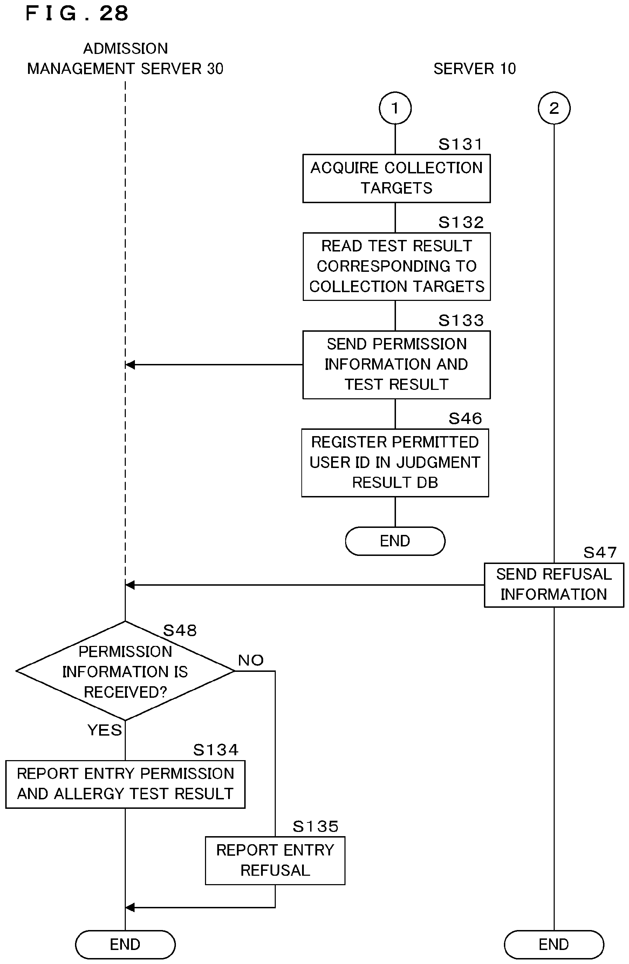

FIG. 28 is a flowchart of one example of a judgment processing procedure according to the eighth embodiment. The processing in FIG. 28 is obtained by adding steps S131-S133 in place of step S45, by adding step S134 in place of step S49, and by adding step S135 in place of steps S50-S51 in the processing in FIG. 11. The description as to the same steps as those in FIGs. 10 and 11 are not repeated. FIG. 28 does not illustrate steps S31-S44 in FIG. 10.

The control unit 21 of the user terminal 20 and the control unit 31 of the admission management server 30 according to the present embodiment perform similar processing to steps S31-S44 in FIG. 10. If determining that the test result of the user satisfies the requirement obtained at step S38 at step S40 (S40: YES), the control unit 11 of the server 10 acquires the test items (collection targets) that are to be collected at the time of entry for the event or the facility (S131). Specifically, if having received the facility ID, the control unit 11 reads the collection targets corresponding to the facility ID from the facility DB 12c. With respect to the event ID as well, if the collection targets have been registered in the event DB 12b, the control unit 11 may read the collection targets corresponding to the event ID from the event DB 12b.

The control unit 11 then reads the test result of the authenticated user from the user DB 12a for the test item corresponding to each of the read collection targets (S132). If reading "allergy test for milk" as a collection target, for example, the control unit 11 reads the allergy test result for milk from the allergy test results stored in the user DB 12a. The control unit 11 sends the permission information indicating that this user is permitted entry and the read allergy test result of the user to the admission management server 30 (S133).

If determining that the permission information is received from the server 10 (S48: YES), the control unit 31 of the admission management server 30 reports permission of the entry to the facility (store) and the allergy test result of the user through the report unit 37 or the display unit 35 (S134). In contrast, if determining that the permission information is not received from the server 10 (S48:NO), the control unit 31 reports refusal of the entry to the facility (store) through the report unit 37 or the display unit 35 (S135). This allows the admission management server 30 to judge whether or not the user is permitted entry based on the test results registered in the server 10 and acquire the allergy test result for the user from the server 10, when the user enters the facility.

The configuration according to the present embodiment can be applied to the information processing system 100 using the gate device 30a installed at the boarding gate of an airport, for example, other than the information processing system 100 using the gate device 30a installed at the entrance of a store such as a restaurant or the like. In this case, when a user boards on an airplane after passing through the boarding gate at an airport, it is possible to judge whether or not the user is permitted boarding based on the test results registered on the server 10 and acquire the allergy test result of the user. Thus, when food containing allergens is provided as in-flight meals, safe in-flight meals can be provided by using the results of the allergy test for each user.

In the present embodiment, the effect similar to each of the embodiments described above can be obtained. In the present embodiment, collection of the allergy test results as well as judgment as to whether or not the user is permitted entry are made possible when the user enters the facility (store). Thus, in a store where products that contain allergens for food allergies are offered such as a restaurant, collecting the user's allergy test results when the user enters the store can offer allergy-friendly products. In the present embodiment as well, the modification appropriately described in each of the above-mentioned embodiments is applicable. In addition, the configuration of the present embodiment can be applied to the information processing system 100 of the second to seventh embodiments, and can produce a similar effect even if it is applied to the information processing system 100 of the second to seventh embodiments.

### Ninth Embodiment

The information processing system will be described that charges based on the number of test items used for judging whether or not the user is permitted attendance or entry or the number of users for whom judgment as to whether or not the user is permitted attendance or entry is performed. Since the information processing system 100 according to the present embodiment can be implemented with the same devices as the information processing system 100 of the first embodiment, the detailed descriptions of their configurations will not be repeated.

In the information processing system 100 according to the present embodiment, each device executes processing similar to the ones in FIG. 6 and FIG. 8. Accordingly, in the present embodiment as well, the event information input via the registration terminal 40 is registered in the server 10, and a code requested via the user terminal 20 is issued by the server 10 and received by the user terminal 20. The devices each perform the same processing as shown in FIGs. 10 and 11. Thus, it is possible to perform judgment as to whether or not the user is permitted to attend an event or enter a facility based on the test result registered in the server 10 when the user passes through the entrance passage of the gate device 30a.

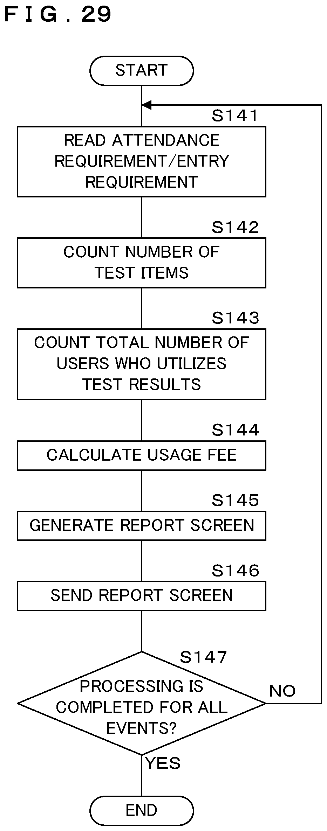

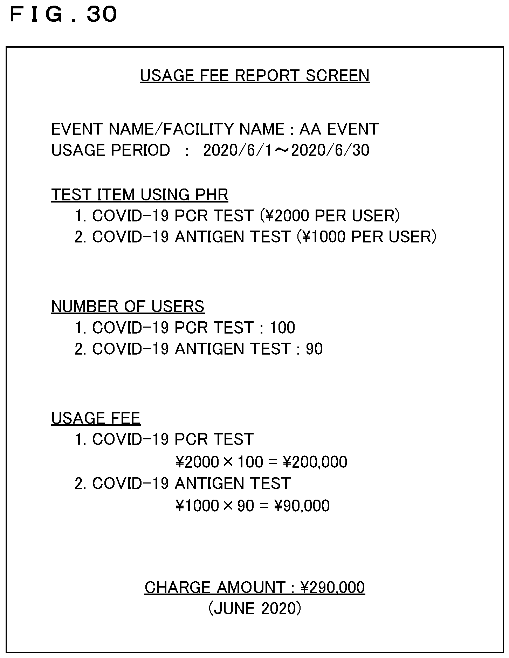

The server 10 according to the present embodiment executes calculation processing of a usage fee for each event or each facility while executing the respective processing described in the first embodiment. FIG. 29 is a flowchart of one example of a procedure of usage fee calculation processing while FIG. 30 is a schematic diagram illustrating a screen example. The following processing is implemented by the control unit 11 according to the control program 12P stored in the storage unit 12 of the server 10. Part of the following processing may be implemented with dedicated hardware circuitry.

In the information processing system 100 according to the present embodiment, the control unit 11 of the server 10 reads an attendance requirement or an entry requirement for one event or for one facility from the event DB 12b or the facility DB 12c, respectively when the timing of calculating a usage fee has come (S141). The timing of calculating a usage fee may be set to a time after the end of the event or after a lapse of a predetermined time period, for example. The control unit 11 counts the number of test items included in the attendance requirement or entry requirement based on the read attendance requirement or entry requirement (S142). It is noted that the number of test items included in the attendance requirement or entry requirement is the number of test items (the number of desired test items) to be used when attendance restrictions or entry restrictions are imposed. The control unit 11 of the server 10 has received the event information or facility information from the registration terminal 40 and registered it in the event DB 12b or facility DB 12c. The control unit 11 thus has obtained the desired test items that are desired for use when the admission management system imposes attendance restrictions or entry restrictions, in association with each event or each facility. It is noted that the number of test items included in each of the attendance requirements or each of the entry requirements may be registered in the event DB 12b or facility DB 12c in association with the event ID or facility ID. In this case, the control unit 11 may merely read the number of test items from the event DB 12b or the facility DB 12c.

The control unit 11 then counts the total number of the number of permitted users and the number of refused users (number of users who utilizes the test results) that are registered in the judgment result DB 12d for this event or facility (S143). It is noted that the number of permitted users and the number of refused users or a total number of users who utilize the test results may be stored in association with the event ID or facility ID in the judgment result DB 12d. If the number of permitted users and the number of refused users are already stored, the control unit 11 reads the respective numbers of users from the judgment result DB 12d to calculate the total number of users. Alternatively, if the total number of users are already stored, the control unit 11 may merely read the total number of users from the judgment result DB 12d.

The control unit 11 then calculates the usage fee for this event or facility based on the number of test items or the number of users who use the test results (S144). For example, a charge for one test item may be set in advance, a charge for one user may be set in advance, or a charge for one user may be set for each test item in advance. The control unit 11 stores the calculated usage fee in association with the event ID or facility ID in the storage unit 12. The control unit 11 then generates a report screen for the calculated usage fee (S145) and sends the generated report screen to the terminal of the event organizer or the person in charge of the facility (S146).

FIG. 30 illustrates an example of a screen display of the report screen. The report screen displays an event name or a facility name, the usage period, a test item used for judging whether or not the user is permitted to attend the event or enter the facility, a unit price of usage per user set to each test item, the number of users who uses the test results of respective test items, a usage fee for each test item and a total charge of the usage fee (charge amount) and the like. By sending such a report screen to the organizer of an event or the person in charge of a facility, it is possible to report the fee for usage of the server 10 in order to impose attendance restrictions on the event or the entry restrictions on the facility.

The control unit 11 determines whether the calculation processing is completed for all the events and facilities for which the usage fee is to be calculated (S147). If the processing is not completed (S147: NO), the control unit 11 returns the processing to step S141 and performs the processing from steps S141 to S146 for the unprocessed events and facilities. If determining that the calculation processing is completed for all of the events and facilities (S147: YES), the control unit 11 ends the calculation processing. As a result of the above-mentioned processing, in the information processing system 100 according to the present embodiment, when a user attends the event or enters the facility, the attendance restrictions or the entry restrictions can be imposed on the user by judging whether or not the user is permitted to attend an event or enter a facility, and a charge (usage fee) for the judgment processing for permission or refusal of attendance or entry can be calculated.

In the present embodiment, the effect similar to each of the embodiments described above can be obtained. Moreover, in the present embodiment, when attendance restrictions on the event or the entry restrictions on the facility are imposed, the usage fee for the judgment processing for judging permission or refusal of attendance or entry based on the test results of the user registered on the server 10 can be calculated and reported. In the present embodiment as well, the modification appropriately described in each of the above-mentioned embodiments is applicable. In addition, the configuration of the present embodiment can be applied to the information processing system 100 of the second to eighth embodiments, and can produce a similar effect even if it is applied to the information processing system 100 of the second to eighth embodiments.

### Tenth Embodiment

The information processing system will be described that guides the user to a different guidance destination depending on the result of judgment as to whether or not the user is permitted to attend an event or to enter a facility based on the health record of the user registered on the server 10 when the user attends the event or enters the facility. Since the information processing system 100 according to the present embodiment can be implemented with the same devices as the information processing system 100 of the first embodiment, the detailed descriptions of their configurations will not be repeated. The admission management server 30 according to the present embodiment includes in the storage unit 32 a guidance detail DB 32b in addition to the configuration of the first embodiment illustrated in FIG. 5.

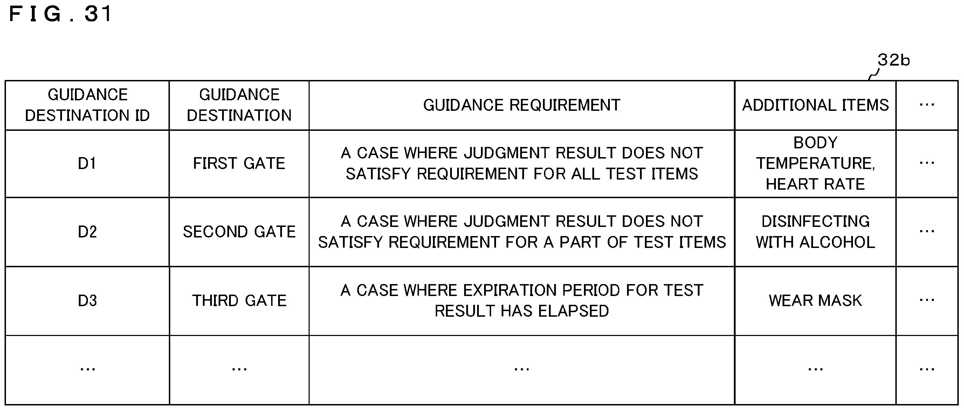

FIG. 31 is a schematic diagram illustrating an example of the configuration of the guidance detail DB 32b. The guidance detail DB 32b stores information on the destinations according to the result of the judgment as to whether or not the attendance or entry is permitted based on the test results of users who attend the event or users who enter the facility. The guidance detail DB 32b in FIG. 31 includes a guidance destination ID column, a guidance destination column, a guidance requirement column, an additional item column and the like. The guidance destination ID column stores the identification information (guidance destination ID) assigned to each of the guidance destinations. The guidance destination column, guidance requirement column and additional item column respectively store information indicating a guidance destination such as a gate number, a requirement set when the user is guided for each guidance destination and information on a test or the like to be additionally conducted for each guidance destination, in association with each guidance destination ID. Examples of the guidance requirement may include a case where the judgment result as to whether or not the user is permitted attendance or entry does not satisfy the requirement for all the test items, a case where it does not satisfy the requirement for a part of the test items, and a case where an expiration period for the test result has elapsed. Examples of the guidance requirement may include a case where the result of the PCR test for COVID-19 is positive, a case where the result of the antigen test for COVID-19 is negative, a case where the user has not been inoculated with a vaccine against COVID-19 (first or second dose), and a case where a predetermined time has elapsed since the user was inoculated with a vaccine against COVID-19. Additional items include the measurement of biometric information such as body temperature, respiratory rate, heart rate and the like, tests for various test items that can be detected by exhalation, checking for wearing a mask and disinfecting hands with alcohol. The guidance destination ID stored in the guidance detail DB 32b is issued and stored by the control unit 31 when each guidance destination is registered. The rest of the information stored in the guidance detail DB 32b is stored by the control unit 31 if the control unit 31 acquires the information on a guidance destination through the input unit (not illustrate), and changed by the control unit 31 if a change instruction is acquired. Examples of the stored contents of the guidance detail DB 32b may be a various information about the guidance destination, not limited to the examples shown in FIG. 31. Moreover, the guidance detail DB 32b may be created by a different terminal such as the terminal of the event organizer or the terminal of the person in charge of the facility, for example. In this case, the admission management server 30 may acquire the guidance detail DB 32b from the different terminal via the network N and store it in the storage unit 32.

In the information processing system 100 according to the present embodiment, each device executes processing similar to the ones in FIG. 6 and FIG. 8. Accordingly, in the present embodiment as well, the event information input via the registration terminal 40 is registered in the server 10, and a code requested via the user terminal 20 is issued by the server 10 and received by the user terminal 20.

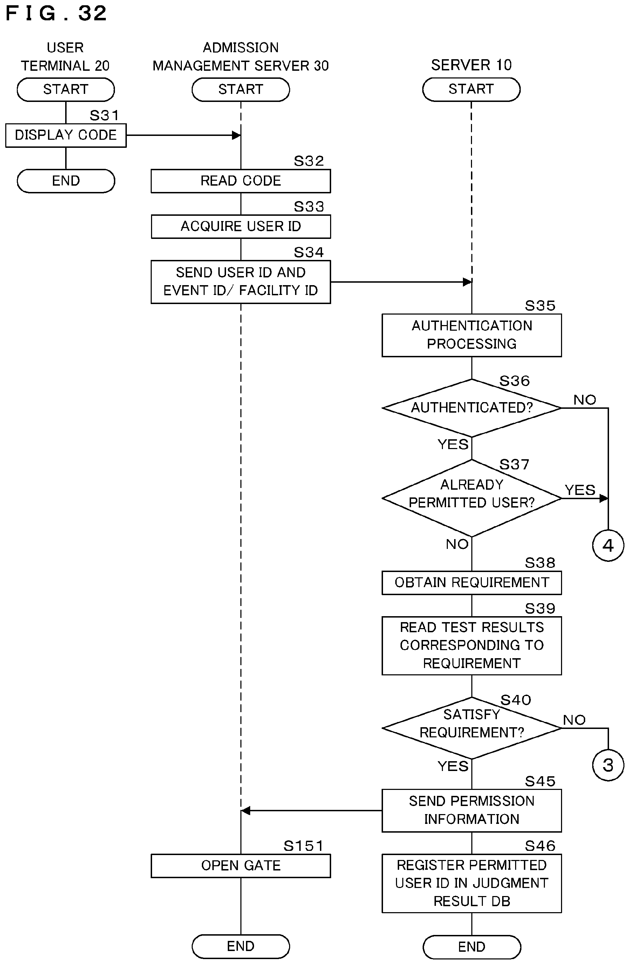

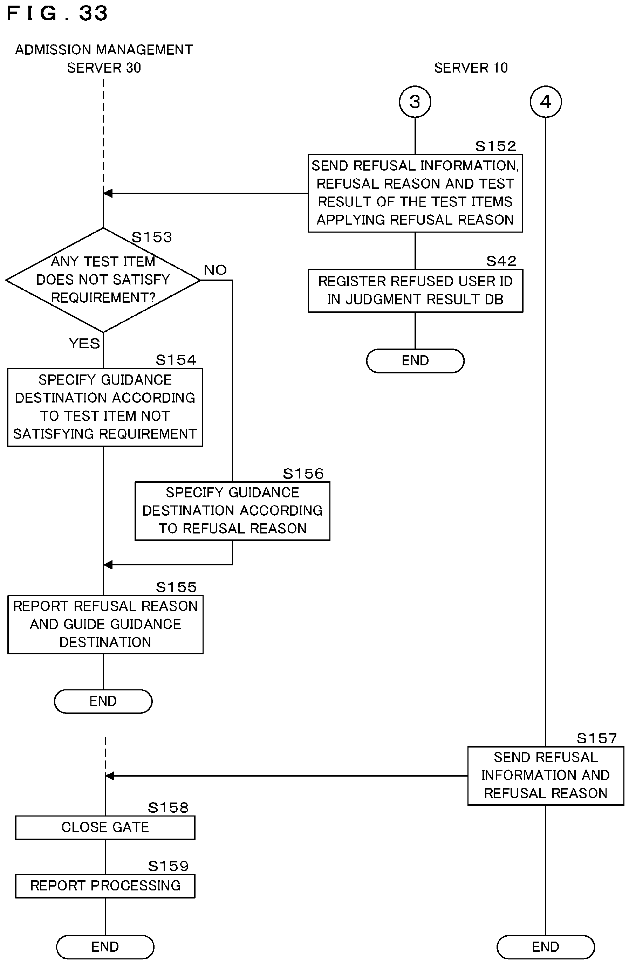

FIGs. 32 and 33 are each a flowchart of one example of the judgment processing procedure according to the tenth embodiment. The processing in FIGs. 32 and 33 are obtained by partially changing steps S41-S51 of the processing in FIGs. 10 and 11. The description as to the same steps as those in FIGs. 10 and 11 are not repeated. The control unit 21 of the user terminal 20, the control unit 31 of the admission management server 30 and the control unit 11 of the server 10 according to the present embodiment perform similar processing to steps S31-S40 in FIG. 10.

If determining that the test result of the user satisfies the attendance requirement or entry requirement at step S40 (S40: YES), the control unit 11 sends the permission information indicating that the user is permitted attendance or entry to the admission management server 30 (S45) and registers the user ID of the user in the permitted user column of the judgment result DB 12d (S46) in association with the event ID or facility ID. If receiving the permission information from the server 10, the control unit 31 of the admission management server 30 opens the gate 38a with the gate opening/closing unit 38 (S151). Here, the control unit 31 may perform report processing of reporting that attendance at the event or entry to the facility is permitted by the report unit 37.

Meanwhile, if determining that the test result of the user does not satisfy the attendance requirement or entry requirement (S40: NO), the control unit 11 sends to the admission management server 30 the refusal information indicating that the user is refused attendance or entry, the reason for refusal and the test result of the test items to which the reason for refusal applies (S152). The control unit 11 then registers the user ID of the user who is refused attendance or entry in the refused user column of the judgment result DB 12d in association with the event ID or facility ID (S42).

If receiving the refusal information from the server 10, the control unit 31 of the admission management server 30 determines whether the reason for refusal is that there is any test item that does not satisfy the attendance requirement or entry requirement (S153). If determining that there is any test item that does not satisfy the attendance requirement or entry requirement (S153: YES), the control unit 31 specifies a guidance destination according to the test item that does not satisfy the attendance requirement or entry requirement (S154). For example, the control unit 31 specifies the gate as the guidance destination corresponding to the reason for refusal and the test result of the user based on the registration contents of the guidance detail DB 32b. For example, if the requirements for all the test items included in the attendance requirement or entry requirement are not satisfied, the control unit 31 specifies a first gate as the guidance destination for this user. Alternatively, the control unit 31 may specify a guidance destination according to the test result for a predetermined test item, and, for example, may specify a predetermined gate as the guidance destination for the user whose antigen test result for the COVID-19 is negative.

Meanwhile, if determining that the reason for refusal is not that there is any test item that does not satisfy the attendance requirement or entry requirement (S153: NO), i.e., if the reason for refusal is that the user has not received a test for the test item set as the attendance requirement or the entry requirement, or that the expiration period of the test result of the user has elapsed, the control unit 31 specifies a guidance destination according to the reason for refusal (S156). For example, the control unit 31 specifies a gate as the guidance destination corresponding to the reason for refusal based on the registration contents in the guidance detail DB 32b. For example, if the expiration period of the test result of the user has elapsed, the control unit 31 specifies a third gate as the guidance destination of this user. The control unit 31 then reports the reason for refusal by the report processing with the report unit 37 and guides the user to the specified guidance destination (S155). For example, the control unit 31 displays a message such as "go to the first gate" on the display unit 35 to guide the user to the first gate. Here, the control unit 31 may open the gate 38a with the gate opening/closing unit 38 to guide the user to the guidance destination. Thus, when the user attends an event or enter a facility, the admission management server 30 can not only impose attendance restrictions or entry restrictions but also guides the user to a different guidance destination depending on the test result of the user or the result of judgment whether or not the user is permitted attendance or entry. This makes it possible to perform an additional test such as measurement of the body temperature as necessary depending on the test result when the user attends an event or enters a facility, which enables attendance restrictions or entry restrictions while more accurately judging the condition of the user.

Meanwhile, if determining that the user is not authenticated at step S36 (S36: NO), or if determining that the user is already permitted (S37: YES), the control unit 11 sends to the admission management server 30 the refusal information indicating that the user is refused to attendance or entry and the reason for refusal (S157). The reason for refusal is here that the user was not authenticated, that a permission has already been issued, or the like. If receiving the refusal information from the server 10, the control unit 31 of the admission management server 30 closes the gate 38a with the gate opening/closing unit 38 (S158) and reports that attendance at the event or entry to the facility is refused by the report processing with the report unit 37 (S159). Here, the control unit 31 may report the reason for refusal received from the server 10.

In the present embodiment, the effect similar to each of the embodiments described above can be obtained. Furthermore, in the present embodiment, depending on the result of the judgment (the reason for refusal) as to whether or not the user is permitted attendance or entry based on the test results of the user, it is possible to guide the user to a different destination and take a different measure. It should be noted that the measure (guidance destination) depending on the test result may be registered for each test item. In this case, even if the tests are conducted for the same infectious disease, a different measure can be taken for each test using a different type of testing method. In the present embodiment as well, the modification appropriately described in each of the above-mentioned embodiments is applicable. In addition, the configuration of the present embodiment can be applied to the information processing system 100 of the second to ninth embodiments, and can produce a similar effect even if it is applied to the information processing system 100 of the second to ninth embodiments.

Each of the above-mentioned embodiments is configured to judge whether or not the user is permitted attendance or entry based on the test result of the user registered in advance on the server 10 when the user attends an event or enters a facility. As another embodiment, the user may previously judge whether or not he/she is permitted attendance or entry based on the test result of his/her own. In this case, since the user can previously know the permittance or refusal of his/her attendance or entry, the user can take a measure such as receiving an additional test to have a permission for attendance or entry and can prepare himself/herself so as to surely attend the event on the date for the event, for example.

### Eleventh Embodiment

An information processing system will be described that imposes passage restrictions on users at various passing points (passing gates) using a vaccination history. Since the information processing system according to the present embodiment can be implemented with the same devices as the information processing system 100 of the first embodiment, the detailed descriptions of their configurations will not be repeated. In the present embodiment, the user DB12a stored in the storage unit 12 of the server 10 is slightly different in configuration from that in the first embodiment illustrated in FIG. 3. In the present embodiment, since the passage restrictions are imposed on a user based on his/her vaccination history, this eliminates the need for setting a passage requirement for each event or for each facility, this also eliminates the need for providing the registration terminal 40, and this eliminates the need for storing the event DB 12b and facility DB 12c in the storage unit of 12 of the server 10. It is noted that the number of vaccinations may be set as a passage requirement for each event or for each facility. In this case, the passage requirement for each event or each facility may be set using the registration terminal 40 and be stored in the event DB 12b and facility DB 12c of the server 10.

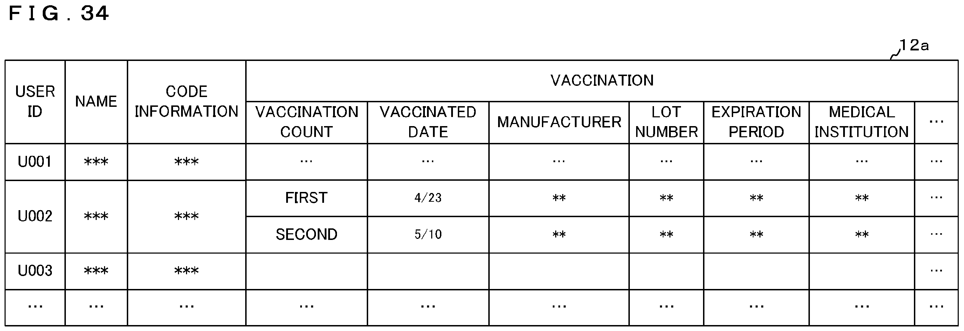

FIG. 34 is a schematic diagram illustrating an example of the configuration of the user DB 12a according to the eleventh embodiment. The user DB 12a according to the present embodiment includes a user ID column, a name column, a code information column, a vaccination column and the like. The code information column stores, in association with a user ID, information (code information) used when a code to be issued for each of the users is generated. It is noted that the code information may include a user ID. The vaccination column stores information on the vaccination history of the user in association with the user ID. The vaccination history information includes information on a vaccinated date, the manufacturer name of an inoculated vaccine, the type, the lot number, the expiration period and a medical institution where the vaccine is received and the like for each vaccination count (how many doses received in the past). The information on an inoculation institution includes the name, the location (e.g., country, prefecture) of the medical institution and the like. It is noted that the vaccination history information may be deleted by the control unit 11 after a predetermined expiration period has elapsed from the vaccinated date, for example. Having been provided with an expiration flag in the user DB 12a, the control unit 11 may be configured to change the expiration flag from 0 to 1 if a predetermined expiration period has elapsed from the vaccinated date.

In the information processing system 100 according to the present embodiment, the devices each perform the same processing as shown in FIG. 8. In the present embodiment, at step S26 in FIG. 8, the control unit 11 of the server 10 reads code information corresponding to the user ID received from the user terminal 20 from the user DB 12a and generate a code based on the read code information. In the present embodiment as well, the control unit 11 (first output unit) of the server 10 issues a code that is requested via the user terminal 20 and outputs the code to the user terminal 20, to provide the user with the code. It is noted that the code issued using the code information associated with the user is provided to the user terminal 20 in the present embodiment.

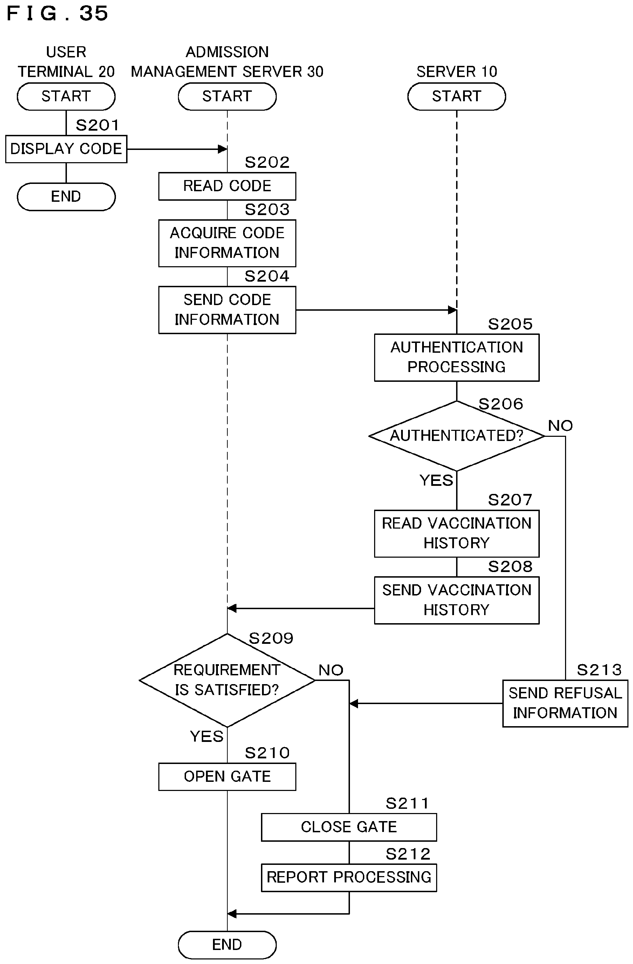

FIG. 35 is a flowchart of one example of a judgment processing procedure according to the eleventh embodiment. FIG. 35 shows the processing performed by the user terminal 20 on the left, the processing performed by the admission management server 30 at the center and the processing performed by the server 10 on the right. In the following processing, two doses of the vaccine within six months are assumed to be set as a passage requirement. The vaccine can be, for example, a vaccine against COVID-19. In the present embodiment as well, if receiving an instruction to execute the PHR application 22AP and to display a code via the input unit 24, the control unit 21 of the user terminal 20 activates the PHR application 22AP and displays the code received from the server 10 on the display unit 25 (S201). By holding the user terminal 20 with the code displayed over the code reader 36 of the gate device 30a, the user causes the code reader 36 to read the code.

The control unit 31 of the admission management server 30 reads the code with the code reader 36 (S202) and acquires code information (S203). The control unit 31 sends the acquired code information to the server 10 (S204) and requests the server 10 to send the vaccination history of this user. When receiving a request to send a vaccination history from the admission management server 30, the control unit 11 (acquisition unit) of the server 10 acquires the code information read by the admission management server 30 and performs authentication processing based on the code information acquired from the admission management server 30 (S205). The control unit 11 here determines whether or not the code information acquired from the admission management server 30 is registered in the user DB 12a and determines the user as a legitimate user if it is registered. The control unit 11 (readout unit and second output unit) determines whether or not the user is authenticated as a legitimate user (S206), and reads the vaccination history information of the authenticated user from the user DB 12a (S207) if determining that the user is authenticated (S206: YES) and sends the read vaccination history information to the admission management server (S208).

When acquiring the vaccination history information from the server 10, the control unit 31 of the admission management server 30 determines whether or not the passage requirement is satisfied based on the acquired vaccination history information (S209). The control unit 31 here determines whether or not this user has received two doses of the vaccine within six months based on the vaccination history information. If determining that the passage requirement is satisfied (S209: YES), i.e., if this user has received two doses of the vaccine within six months, the control unit 31 opens the gate 38a with the gate opening/closing unit 38 (S210). The control unit 31 here may perform report processing of reporting that passing is permitted with the report unit 37.

If determining that the passage requirement is not satisfied (S209: NO), i.e., if this user has not received two doses of the vaccine, or if this user has received two doses of the vaccine, but the expiration period for the vaccine (six months, for example) has elapsed, the control unit 31 closes the gate 38a with the gate opening/closing unit 38 (S211) and reports that passing is refused by the report processing with the report unit 37 (S212). The report processing here is for reporting the reason for refusal of passing and makes a report such as "two doses of the vaccine have not been received" or "the expiration period for the vaccine has elapsed." This allows the user to know that the passage requirement related to the vaccination is not satisfied while this allows the admission management server 30 to report to the user and the event organizer or the person in charge of the facility staying nearby that the user is refused to pass.

If determining that the user cannot be authenticated as a legitimate user at step S206 (S206: NO), the control unit 11 of the server 10 sends refusal information indicating that the user is refused to pass to the admission management server 30 (S213). In this case as well, the control unit 31 of the admission management server 30 closes the gate 38a with the gate opening/closing unit 38 (S211) and reports that passing is refused by the report processing with the report unit 37 (S212). The report processing here is for reporting that the authentication fails and reports "let me read correct code," for example. This allows the user to understand that an erroneous code is read by the admission management server 30.

As a result of the above-mentioned processing, in the information processing system 100 according to the present embodiment, judgment as to whether or not the passage requirement is satisfied is performed based on the vaccination history of the user registered on the server 10 when the user passes through the passing gate. Depending on whether or not the vaccination history of the user satisfies the passage requirement, passing through the passing gate is permitted or refused. Thus, the vaccination history of each of the users registered on the server 10 can be employed for the passage restrictions to be imposed on the users by the admission management system. For example, if vaccination is mandated at a time of entrance to and exit from a country or a designated area, a passing examination can be cleared by using the vaccination history of each of the users registered on the server 10.

In the present embodiment, the judgment as to whether or not the vaccination history of the user satisfies the passage requirement may be performed not only by the admission management server 30 but also by the server 10. In this case, after reading the vaccination history of the user from the user DB 12a, the server 10 determines whether or not the read vaccination history satisfies the passage requirement and sends the determination result to the admission management server 30. It is noted that the passage requirement may be registered in advance in the server 10 or may be acquired from the admission management server 30 by the server 10. In addition, the server 10 may determine whether or not the user is permitted to pass depending on whether or not the vaccination history satisfies the passage requirement and may send the determination result to the admission management server 30. Furthermore, the user DB12 may include information related to the test result of the infectious disease (test date, test results, test location and the like) in addition to various information on the vaccination. In this case, the test result of the infectious disease is read at the same timing as S207 in FIG. 35 and sent to the admission management server 30 along with the vaccination history. The passage requirement may include that the test result of the infectious disease (the test result of the infectious disease received within a predetermined period before passing, for example) is negative in addition to the above-mentioned passage requirement for the vaccination.

In the present embodiment, the effect similar to the above-mentioned respective embodiments can be obtained. In the present embodiment, it is possible to judge whether or not the user is permitted to pass (attend an event or enter a facility) depending on the vaccination history of the user. For example, if vaccination against COVID-19 is mandated at a time of entrance to and exit from a country or a designated area, a passing examination is cleared based on the vaccination history against the COVID-19 registered in the server 10. In the present embodiment as well, the modification appropriately described in each of the above-mentioned embodiments is applicable. In addition, the configuration of the present embodiment can be applied to the information processing system 100 of the second to tenth embodiments, and can produce a similar effect even if it is applied to the information processing system 100 of the second to tenth embodiments.

In the present embodiment, at step S26 in FIG. 8, the control unit 11 of the server 10 is configured to read from the user DB 12a the code information corresponding to the user ID received from the user terminal 20 and generate a code based on the read code information as described above. Though the configuration is not limited the above description, the code to be generated may include information on the vaccination history and/or information on the test result of an infectious disease in addition to the code information. In this case, the control unit 11 sends a screen including a code such as a QR code or the like and the information on a vaccination history and/or the information on the test result of an infectious disease in text format to the user terminal 20. Then, when reading the code on the screen, the admission management server 30, which has stored the passage requirement, acquires the code information included in the code as well as the information on the vaccination history and/or the information on the test result of the infectious disease to determine whether or not they satisfy the passage requirement. This allows the admission management server 30 alone to judge whether or not the user is permitted to pass without going through the server 10.

### Twelfth Embodiment

An information processing system will be described that is configured to perform processing according to a vaccination history and the details of the test result of the infectious disease upon passage restrictions when a user passes various passing points (passing gates). Since the information processing system 100 according to the present embodiment can be implemented with the same devices as the information processing system 100 of the first embodiment, the detailed descriptions of their configurations will not be repeated. In the present embodiment, the admission management server 30 stores a corresponding processing DB 32c and a passage management DB 32d in the storage unit 32 in addition to the configuration of the first embodiment illustrated in FIG. 5. The corresponding processing DB 32c and the passage management DB 32d may be stored in another storage device connected to the admission management server 30, or may be stored in another storage device with which the admission management server 30 can communicate over a network N. In the present embodiment as well, the registration terminal 40 is not necessarily provided, and the even DB 12b and the facility DB 12c are not necessarily stored in the storage unit 12 of the server 10. It is noted that a requirement based on the number of vaccinations and/or the test result of the infectious disease may be set as a passage requirement for each event or each facility.

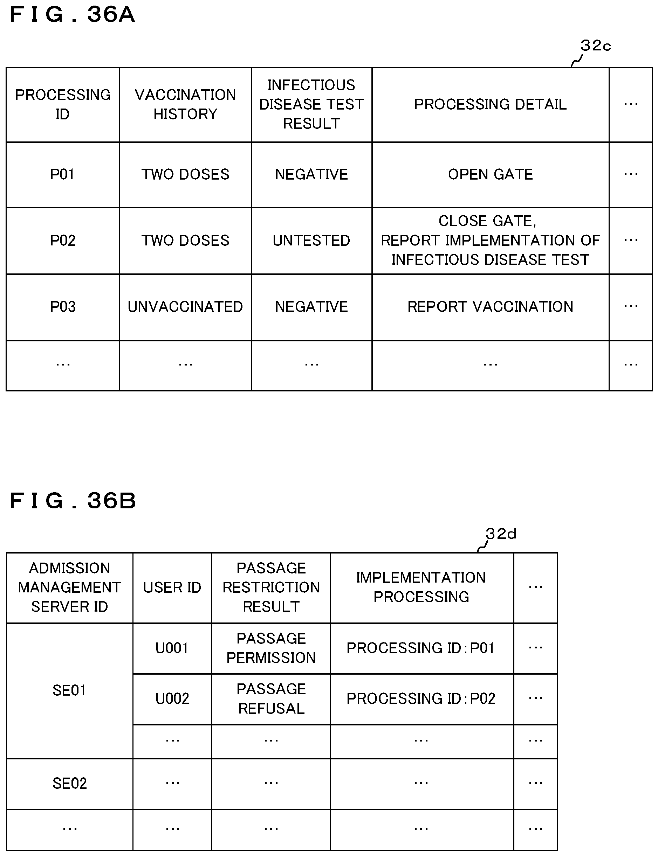

FIG. 36A is a schematic diagram illustrating an example of the configuration of the corresponding processing DB 32c. The corresponding processing DB 32c stores information on the processing to be performed depending on the vaccination history and the test result of the infectious disease. Specifically, the corresponding processing DB 32c includes a processing ID column, a vaccination history column, an infectious disease test result column, a processing detail column, etc. The processing ID column stores identification information (processing ID) to identify each processing. The vaccination history column, for example, stores requirement information on the vaccination history of the COVID-19, and specifically includes two doses, one dose, and unvaccinated. The infectious disease test result column, for example, stores requirement information on the PCR test result for the COVID-19, and specifically includes negative, positive, and untested. The processing detail column stores information on the processing to be executed according to each requirement of the vaccination history and each requirement of the test result of the infectious disease. The processing detail includes opening or closing the gate by the gate device 30a, reporting a predetermined message by the report unit 37 and displaying a predetermined message by the display unit 35. The corresponding processing DB 32c thus configured registers the processing to be executed at a passing gate according to the vaccination history and the test result of the infections disease. The details of the corresponding processing DB 32c are already set and stored, and are appropriately changeable.

FIG. 36B is a schematic diagram illustrating an example of the configuration of the passage management DB 32d. The passage management DB 32d stores information on the result of passage restrictions imposed on the user by the admission management server 30. Specifically, the passage management DB 32d includes an admission management server ID column, a user ID column, a passage restriction result column, and an implementation processing column. The admission management server ID column is an ID assigned to the admission management server 30 that imposes passage restrictions on the user. The admission management server ID column may be configured to store a gate ID assigned to the gate device 30a in addition to the ID of the admission management server 30. The user ID column stores the user ID of the user who is judged to permit or refused to pass by the admission management server 30. The passage restriction result column stores information indicating the result of the passage restrictions performed by the admission management server 30 and stores a passage permission or a passage refusal, for example. The implementation processing column stores the processing details executed at the time of the passage restrictions and stores the processing ID of each processing registered in the corresponding processing DB 32c, for example. The passage management DB 32d thus configured can accumulate whether or not each user is permitted to pass and the details of the processing implemented at the gate device 30a at that time, as a result of the passage restrictions by the admission management server 30. The passage management DB 32d may be configured to store the vaccination history and the test result of the infectious disease for each user.

In the information processing system 100 according to the present embodiment, the devices each perform the same processing as shown in FIG. 8. In the present embodiment as well, the control unit 11 of the server 10 generates a code based on the code information corresponding to the user ID received from the user terminal 20 at step S26 in FIG. 8. In the present embodiment as well, a code based on the code information associated with the user requested via the user terminal 20 is issued by the server 10 and received by the user terminal 20. It is noted that the control unit 11 of the server 10 operates as a first output unit that outputs a code generated from the code information associated with the vaccination history of the user who requests a code to the user terminal of the user as a request source.

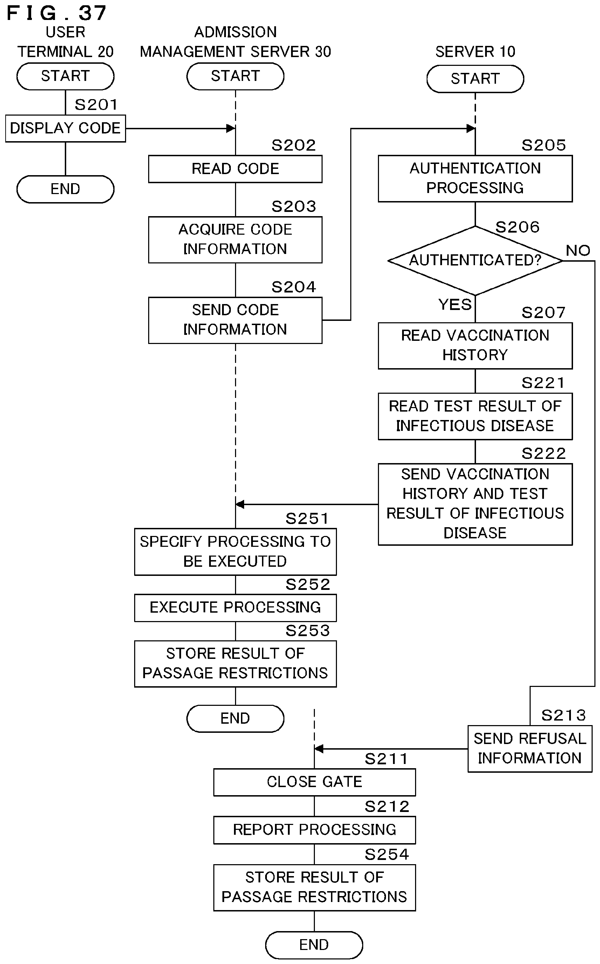

FIG. 37 is a flowchart of one example of a judgment processing procedure according to the twelfth embodiment. The processing in FIG. 37 is obtained by adding steps S221-S222 in place of step S208, by adding steps S251-253 in place of steps S209-210, and by adding step S254 after step S212 of the processing in FIG. 35. The descriptions as to the same steps as those in FIG 35 are not repeated. The control unit 21 of the user terminal 20 and the control unit 31 of the admission management server 30 and the control unit 11 of the server 10 according to the present embodiment perform similar processing to those at steps S201 to S207 in FIG. 35. The control unit 11 of the server 10 reads the information on the vaccination history of the authenticated user from user DB 12a (S207), reads the information on the test result of the infectious disease from the user DB 12a (S221), and sends the information on the vaccination history and the information on the test result of the infectious disease that are read to the admission management server 30 (S222). The control unit 11 of the server 10 thus operates as a second output unit that outputs to the admission management server 30 the vaccination history and the test result of the infectious disease of the user whose code has been read by the admission management server 30.

In the present embodiment, if acquiring the information on the vaccination history and the information on the test result of the infectious disease from the server 10, the control unit 31 of the admission management server 30 specifies the processing to be executed by the gate device 30a based on the information on the vaccination history and the information on the test result of the infectious disease that are acquired (S251). Here, the control unit 31 specifies a vaccination history and a test result of the infectious disease that match the information on the vaccination history and the information on the test result of the infectious disease acquired from the server 10 based on the stored contents of the corresponding processing DB 32c, and specifies the processing details corresponding to the vaccination history and the test result of the infectious disease that are specified.

The control unit 31 (execution unit) instructs the gate device 30a to execute the specified processing, and executes the specified processing at the gate device 30a (S252). For example, if specifying opening of the gate as a processing to be executed, the control unit 31 instructs the gate device 30a to open the gate, and the gate device 30a opens the gate 38a with the gate opening/closing unit 38 to permit the user to pass through the gate. Moreover, if specifying closing of the gate and making a report of implementation of the infectious disease test as a processing to be executed, the control unit 31 instructs the gate device 30a to close the gate and reports the implementation of the infectious disease test. In this case, the gate device 30a closes the gate 38a with the gate opening/closing unit 38 to refuse user's passing, and reports the necessity of implementation of an infectious disease test by the report processing with the report unit 37.

The control unit 31 stores the result of the executed passage restrictions in the passage management DB 32d (S253). Specifically, the control unit 31 stores, in association with the ID of the admission management server 30, the user ID of the user on which the passage restrictions are imposed, the result of the passage restrictions indicating whether or not the user is permitted or refused to pass and the processing implemented when the passage restrictions are imposed in the passage management DB 32d. The processing performed when the passage restrictions are imposed may include the processing ID of each processing registered in the corresponding processing DB 32c, for example. It is noted that the control unit 31 may store the code information acquired at step S203, the vaccination history and the test result of the infections disease that are received from the server 10 and the like in the passage management DB 32d. According to the above-described processing, the admission management server 30 can not only impose passage restrictions on the user at the gate, but also make a report of various messages by performing processing according to the vaccination history and the test result of the infectious disease of the user. In addition, as a result of imposing passage restrictions, the admission management server 30 can accumulate the permission or refusal of the user passing and the processing performed on each user by the gate device 30a and can also store information on the passage restrictions for each user. It is noted that the admission management server 30 may be configured to send such information on the result of the passage restrictions to the server 10, and the serve 10 may be configured to store the information on the passage restrictions for each user in the user DB 12a, for example.

Meanwhile, if determining that the user is not authenticated at step S206 (S206: NO), the control unit 11 of the server 10 sends the refusal information to the admission management server 30 (S213). In this case, the control unit 31 of the admission management server 30 closes the gate 38a with the gate opening/closing unit 38 (S211) and reports that passing is refused by the report processing with the report unit 37 (S212). The control unit 31 then stores the result of the executed passage restrictions in the passage management DB 32d (S254). Here, the control unit 31 stores, in association with the ID of the admission management server 30, the user ID of the user on whom the passage restrictions are imposed, the result of the passage restrictions indicating that passing is refused and the processing implemented when the passage restrictions are imposed (specifically, closing of the gate) in the passage management DB 32d. The control unit 31 here may also store the code information or the like of the user obtained at step S203 in the passage management DB 32d. Thus, the admission management server 30 can accumulate information on the result indicating that the user is refused to pass because not being unauthenticated.

In the present embodiment, the effect similar to each of the embodiments described above can be obtained. Additionally, in the present embodiment, processing according to the vaccination history and the test result of the infectious disease of the user can be performed, which enables a different measure for each user. In the present embodiment as well, the modification appropriately described in each of the above-mentioned embodiments is applicable. In addition, the configuration of the present embodiment can be applied to the information processing system 100 of the second to tenth embodiments, and can produce a similar effect even if it is applied to the information processing system 100 of the second to tenth embodiments.

It is to be understood that the embodiments disclosed here are illustrative and not restrictive in all respects. The scope of the present invention is defined by the appended claims, not by the above-mentioned meaning, and all changes that fall within the meanings and the bounds of the claims, or equivalence of such meanings and bounds are intended to be embraced by the claims.

### [Description of Reference Numerals]

- 10: server
- 11: control unit
- 12: storage unit
- 13: communication unit
- 20: user terminal
- 30: admission management server
- 31: control unit
- 32: storage unit
- 33: communication unit
- 34: gate device communication unit
- 36: code reader
- 37: report unit
- 38: gate opening/closing unit
- 39: biometric information acquisition unit
- 40: registration terminal
- 12a: user DB
- 12b: event DB
- 12c: facility DB
- 30a: gate device
- 32a: ticket DB
- 32b: guidance detail DB
- 36b: IC card reader
- 36c: camera
- 38a: gate

## Claims

1. An information processing method, wherein
a health record is stored in association with user identification information for each user in a database, and
the information processing method comprises processing of:
acquiring the user identification information read by a dispersed management system;
reading from the database the health record corresponding to the user identification information acquired; and
outputting the health record read to the management system.

2. The information processing method according to claim 1, wherein
a passage requirement for the passing point based on the health record is stored, for each passing point managed for passage of the user by the management system, in a database in association with passing point identification information, and
the method comprises the processing of:
acquiring the user identification information read by the management system and the passing point identification information;
reading a health record according to a passage requirement corresponding to the passing point identification information acquired, out of the health record in the database corresponding to the user identification information acquired;
judging whether or not the passage requirement for the passing point is satisfied based on the health record read; and
outputting a judgment result to the management system.

3. The information processing method according to claim 2, wherein
user identification information of a user judged to satisfy the passage requirement for the passing point is stored in a database in association with the passing point identification information, and
the method comprises the processing of
outputting a judgement result indicating that the passage requirement for the passing point is not satisfied to the management system if the user identification information acquired is stored in the database in association with the passing point identification information acquired.

4. The information processing method according to any one of claims 1 to 3 comprising the processing of
outputting the health record to the management system that manages opening or closing of a gate.

5. The information processing method according to any one of claims 1 to 4, wherein
the health record is selected from: a test result for a presence or an absence of infection caused by an infectious disease, of an antibody, of vaccination and of an allergy disease caused by an allergen; a medical history; and a medication history.

6. The information processing method according to any one of claims 1 to 5 comprising the processing of
causing the management system to read authentication information of the user, and
causing the management system to perform authentication of the user based on the authentication information.

7. The information processing method according to any one of claims 1 to 6 comprising the processing of
generating a code including the user identification information and ticket information required for passage of a passing point managed by the management system; and
outputting the code generated to a user terminal of the user.

8. The information processing method according to any one of claims 1 to 7, wherein
ticket information required for passage of a passing point managed by the management system is associated with user identification information in a conversion table, and
the method further comprises the processing of:
acquiring converted user identification information that is converted through the conversion table in association with the ticket information read by the management system;
reading from the database a health record corresponding to the converted user identification information acquired; and
outputting information related to the health record read to the management system from which ticket information to be converted is read.

9. An information processing method comprising processing of:
acquiring user identification information of a user;
acquiring, based on the user identification information acquired, a health record corresponding to the user identification information from a server having a database that stores health record in association with user identification information for each user; and
judging whether or not the user is permitted to pass a passing point based on the health record acquired.

10. An information processing method, wherein
a health record is stored in association with user identification information for each user in a database, and
the information processing method comprises processing of:
reading a health record corresponding to the user identification information form the database;
generating a code including the health record read and the user identification information; and
outputting the code generated to a user terminal of the user.

11. The information processing method according to claim 10 comprising the processing of reading a health record of the user according to a passage requirement for a passing point out of the health record corresponding to the user identification information.

12. An information processing method, wherein
a passage requirement for a passing point based on a health record of a user is stored, and
the information processing method comprises the processing of
acquiring a health record read from a code including user identification information and the health record of a user;
judging whether or not the passage requirement is satisfied based on the health record acquired; and
judging whether or not the user is permitted to pass through the passing point based on a judgment result.

13. The information processing method according to claim 12, wherein
user identification information of the user permitted to pass through the passing point is stored in a database, and
the method comprises the processing of:
acquiring the user identification information read from the code; and
refusing the user passing through the passing point if the user identification information acquired is stored in the database.

14. An information processing method executed by an information processing system including a server having a database storing a health record in association with user identification information for each user and a dispersed management system, wherein
the management system
acquires the user identification information, and
outputs the user identification information acquired to the server;
the server
reads from the database a health record corresponding to the user identification information acquired from the management system, and
outputs the health record read to the management system;
the management system
judges whether or not the user is permitted to pass through a passing point based on a health record corresponding to the user identification information acquired from the server.

15. An information processing method, wherein
a health record for each user is stored in a database, and
the information processing method comprises processing of
outputting a code associated with the health record to a user terminal of the user;
acquiring code information obtained by a dispersed management system reading the code;
reading from the database a health record corresponding to the code information acquired; and
outputting the health record read to the management system.
